# EUROPEAN PATENT APPLICATION

(11) **EP 3 561 049 A1**
(43) Date of publication of application: **30.10.2019**
(21) Application number: 17883408.1
(22) Date of filing: 27.11.2017
(51) Int. Cl.: C12N 1/21, C12N 15/70, C12N 9/02, C12N 9/04, C12P 17/16

(54) **GENETICALLY ENGINEERED BACTERIUM OF COEXPRESSING CYCLOHEXANONE MONOOXYGENASE AND ISOPROPANOL DEHYDROGENASE AND USE THEREOF**

(30) Priority: 23.12.2016 CN 201611202462
(71) Applicant: Zhejiang Jingxin Pharmaceutical Co., Ltd., Shaoxin, Zhejiang 312500 (CN); Shanghai Jingxin Biology&Pharmaceutical Co., Ltd., Shanghai 201203 (CN)
(72) Inventor: JIN, Shengfang, Zhejiang 312500 (CN); ZHANG, Minjie, Zhejiang 312500 (CN); HUANG, Yue, Zhejiang 312500 (CN)
(74) Representative: Proi World Intellectual Property GmbH
(86) International application number: PCT/CN2017/113011
(87) International publication number: WO 2018/113476

(57) **Abstract**

The present invention provides a genetically engineered strain for co-expressing cyclohexanone monooxygenase and isopropanol dehydrogenase and application thereof, and the genetically engineered strain constructed can catalytically convert high-concentration omeprazole sulfide into esomeprazole.

## Description

### Technical Field

The invention belongs to the field of genetic engineering and fermentation engineering, and relates to a genetically engineered strain for co-expressing cyclohexanone monooxygenase and isopropanol dehydrogenase and application thereof, in particular to a genetically engineered strain with high level of co-expression of cyclohexanone monooxygenase and isopropanol dehydrogenase, and the application in the preparation of chiral sulfoxide compounds, particularly in the preparation of esomeprazole.

### Background

Proton pump inhibitors (PPIs) are a class of drugs widely used in clinical practice for the treatment of gastric ulcers in recent decades. Traditional proton pump inhibitors are mainly chiral sulfoxides, such as esomeprazole, levopantoprazole, and dexlansoprazole. Wherein, esomeprazole is omeprazole in the S configuration, with chemical name of 5-methoxy-2-((S)-((4-methoxy-3, 5-dimethyl-2-pyridyl)methyl)sulfinyl)-1H-benzimidazole, CAS accession number 119141-88-7, and the chemical structure shown in formula I below; and chemical name of omeprazole is 5-methoxy-2-[(4-methoxy-3,5-dimethyl-2-pyridyl)sulfinyl]-3H-benzimidazole, with CAS accession number 73590-58-6, and the chemical structure shown in formula II below.

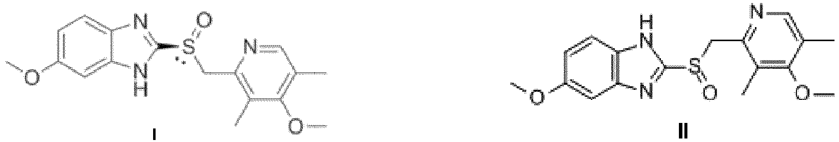

Compared with omeprazole, esomeprazole has the advantages of low liver first-pass effect, high blood concentration, long plasma half-life, high bioavailability, small interaction between drugs, and strong and long-lasting drug efficacy, especially suitable for special populations such as the elderly, and patients with renal insufficiency and mild to moderate hepatic insufficiency. In addition, esomeprazole is also indicated for long-term maintenance therapy in patients with cured esophagitis to prevent recurrence, and can eradicate helicobacter pylori, heal the duodenal ulcer associated with helicobacter pylori infection, and prevent recurrence of peptic ulcer associated with helicobacter pylori in combined with appropriate antibacterial therapy. Esomeprazole ranks fifth in the world in 2004 with its outstanding quality, with sales of $4.3 billion.

At present, there are two main methods for bio-enzymatic synthesis of prazoles: one is to perform whole cell catalysis with wild strains, and the other is to perform catalysis with genetically engineered strain carrying monooxygenase, plus coenzyme or coenzyme regeneration system. A series of strains which can be used for preparing prazole compounds by biological oxidation reaction were screened in US 5840552A, with good catalytic chiral selectivity, and more than 99% product ee value, but the disadvantage is that the product concentration in ppm level is relatively low. CN 102884178 A discloses a non-naturally occurring monooxygenase for the catalyzed synthesis of prazole compounds with higher concentration, however, the method also requires a cofactor and an enzyme for the regeneration of the cofactor, thus is not suitable for industrial production as the cumbersome operation procedure, higher cost and relatively low (only 33 g/L) concentration of the substrate which can be catalyzed by one batch reaction.

### Summary of the Invention

In view of the above problems, an object of the present invention is to provide a genetically engineered strain for co-expressing cyclohexanone monooxygenase (CHMO) and isopropanol dehydrogenase (KRED), a construction method, and application in the preparation of chiral sulfoxide compounds, especially in the preparation of esomeprazole.

In order to achieve the above object, the present invention provides a genetically engineered strain for co-expressing cyclohexanone monooxygenase and isopropanol dehydrogenase, the strain comprising a cyclohexanone monooxygenase gene and an isopropanol dehydrogenase gene. Preferably, in the above genetically engineered strain, the gene of cyclohexanone monooxygenase CHMO and the gene of isopropanol dehydrogenase KRED are integrated in the same expression vector such as a plasmid. Wherein, the amino acid sequence of the cyclohexanone monooxygenase is selected from the group consisting of SEQ ID NO. 2, SEQ ID NO. 4, SEQ ID NO. 6, SEQ ID NO. 8, SEQ ID NO. 10, and SEQ ID NO 12; the amino acid sequence of the isopropanol dehydrogenase is selected from the group consisting of SEQ ID NO. 14, SEQ ID NO. 16, SEQ ID NO. 18, SEQ ID NO. 20, SEQ ID NO. 22, and SEQ ID NO 24; the nucleotide sequence of the gene encoding the cyclohexanone monooxygenase is correspondingly selected from the group consisting of SEQ ID NO. 1, SEQ ID NO. 3, SEQ ID NO. 5, SEQ ID NO. 7, SEQ ID NO. 9, and SEQ ID NO. 11 respectively, and the nucleotide sequence of the gene encoding the isopropanol dehydrogenase is correspondingly selected from the group consisting of SEQ ID NO. 13, SEQ ID NO. 15, SEQ ID NO. 17, SEQ ID NO. 19, SEQ ID NO. 21, and SEQ ID NO. 23.

The genetically engineered strain for co-expressing cyclohexanone monooxygenase and isopropanol dehydrogenase can be constructed by the methods of:
(1) using the cyclohexanone monooxygenase gene fragment as a template, performing PCR amplification, inserting the gene fragment into plasmids using restriction endonuclease Nde I and BamH I recognition sites, and transferring the vector obtained after ligation into E. coli Trans-Tl competent cells (provided by TransGen Biotech Co., Ltd.) to construct a recombinant plasmid pETC-n;
(2) using the isopropanol dehydrogenase gene fragment as a template, performing PCR amplification, inserting the gene fragment into the recombinant plasmid pETC-n obtained by the above step (1) using restriction endonuclease BamH I and Xho I recognition sites, and transferring the vector obtained after ligation into E. coli Trans-T1 competent cells to construct a recombinant expression vector pETCK-n;
(3) transforming the recombinant expression vector pETCK-n obtained by the above construction into an expression host, performing the colony PCR using the primer T7/T7term, after verification, and obtaining the genetically engineered strain BL21(DE3)-pETCK-n co-expressing cyclohexanone monooxygenase gene and isopropanol hydrogenase gene.

In the above method, the plasmids of step (1) are selected from the group consisting of Plasmid pET 21a, Plasmid pET 28a, Plasmid pET 22b, Plasmid pETDuet 1, Plasmid pRSFDuet 1, or Plasmid pACYCDuet 1.

The primers T7 and T7term of the step (3) are conventional primers in the art and are commercially available; and the host includes, but is not limited to, *Escherichia coli* BL21 (DE3), *Escherichia coli* BL21 (DE3) plysS or *Escherichia coli* BL21 (DE3) star.

In one embodiment of the present invention, a genetically engineered strain for co-expressing cyclohexanone monooxygenase and isopropanol dehydrogenase was obtained using the above method, the strain comprising a cyclohexanone monooxygenase gene and an isopropanol dehydrogenase gene, wherein the amino acid sequence of the cyclohexanone monooxygenase is as shown in SEQ ID NO. 2, and the amino acid sequence of the isopropanol dehydrogenase is as shown in SEQ ID NO. 16; the nucleotide sequence of the gene encoding the cyclohexanone monooxygenase is as shown in SEQ ID NO. 1, and the nucleotide sequence of the gene encoding the isopropanol dehydrogenase is as shown in SEQ. ID NO.15.

In another embodiment of the present invention, a genetically engineered strain for co-expressing cyclohexanone monooxygenase and isopropanol dehydrogenase was obtained using the above method, the strain comprising a cyclohexanone monooxygenase gene and an isopropanol dehydrogenase gene, wherein the amino acid sequence of the cyclohexanone monooxygenase is as shown in SEQ ID NO. 4, and the amino acid sequence of the isopropanol dehydrogenase is as shown in SEQ ID NO. 14; the nucleotide sequence of the gene encoding the cyclohexanone monooxygenase is as shown in SEQ ID NO. 3, and the nucleotide sequence of the gene encoding the isopropanol dehydrogenase is as shown in SEQ ID NO. 13.

In yet another embodiment of the present invention, a genetically engineered strain for co-expressing cyclohexanone monooxygenase and isopropanol dehydrogenase was obtained using the above method, the strain comprising a cyclohexanone monooxygenase gene and an isopropanol dehydrogenase gene, wherein the amino acid sequence of the cyclohexanone monooxygenase is as shown in SEQ ID NO. 6, and the amino acid sequence of the isopropanol dehydrogenase is as shown in SEQ ID NO. 18; the nucleotide sequence of the gene encoding the cyclohexanone monooxygenase is as shown in SEQ ID NO. 5, and the nucleotide sequence of the gene encoding the isopropanol dehydrogenase is as shown in SEQ ID NO. 17.

In another embodiment of the present invention, a genetically engineered strain for co-expressing cyclohexanone monooxygenase and isopropanol dehydrogenase was obtained using the above method, the strain comprising a cyclohexanone monooxygenase gene and an isopropanol dehydrogenase gene, wherein the amino acid sequence of the cyclohexanone monooxygenase is as shown in SEQ ID NO. 8, and the amino acid sequence of the isopropanol dehydrogenase is as shown in SEQ ID NO. 20; the nucleotide sequence of the gene encoding the cyclohexanone monooxygenase is as shown in SEQ ID NO. 7, and the nucleotide sequence of the gene encoding the isopropanol dehydrogenase is as shown in SEQ ID NO. 19.

In yet another embodiment of the present invention, a genetically engineered strain for co-expressing cyclohexanone monooxygenase and isopropanol dehydrogenase was obtained using the above method, the strain comprising a cyclohexanone monooxygenase gene and an isopropanol dehydrogenase gene, wherein the amino acid sequence of the cyclohexanone monooxygenase is as shown in SEQ ID NO. 10, and the amino acid sequence of the isopropanol dehydrogenase is as shown in SEQ ID NO. 22; the nucleotide sequence of the gene encoding the cyclohexanone monooxygenase is as shown in SEQ ID NO. 9, and the nucleotide sequence of the gene encoding the isopropanol dehydrogenase is as shown in SEQ ID NO. 21.

In another embodiment of the present invention, a genetically engineered strain for co-expressing cyclohexanone monooxygenase and isopropanol dehydrogenase was obtained using the above method, the strain comprising a cyclohexanone monooxygenase gene and an isopropanol dehydrogenase gene, wherein the amino acid sequence of the cyclohexanone monooxygenase is as shown in SEQ ID NO. 12, and the amino acid sequence of the isopropanol dehydrogenase is as shown in SEQ ID NO. 24; the nucleotide sequence of the gene encoding the cyclohexanone monooxygenase is as shown in SEQ ID NO. 11, and the nucleotide sequence of the gene encoding the isopropanol dehydrogenase is as shown in SEQ ID NO. 23.

Further, the present invention also provides a shaking culture method of the genetically engineered strain (BL21(DE3)-pETCK-n) which co-expresses cyclohexanone monooxygenase and isopropanol dehydrogenase, including the following steps:
the genetically engineered strain BL21(DE3)-pETCK-n was inoculated into 3-5 mL of liquid LB medium containing corresponding plasmid resistance, and activated in by shaking at 37°C for 12-36 h; the activated culture was transferred to the fermentation medium at an inoculation amount of 0.5%-5% (v/v), fermented at 25-40°C for 1-3 h, and added with an inducer propyl-β-D-thiogalactopyranoside (IPTG) with final concentration of 0.05-2 g/L, and induced to express at 15-35°C for 2-24 h; the bacterial sludge is obtained through centrifugation, filtration or ultrafiltration of the expression product, and cryopreserved at -80°C.

Wherein the inoculation amount refers to the percentage of the volume of the activated genetically engineered strain added to the volume of the fermentation medium.

In addition, the present invention also provides the application of the genetically engineered strain co-expressing cyclohexanone monooxygenase and isopropanol dehydrogenase in the preparation of chiral sulfoxide compounds, particularly in the preparation of esomeprazole.

Accordingly, the present invention also relates to a method for preparing esomeprazole comprising: adding a genetically engineered strain co-expressing cyclohexanone monooxygenase and isopropanol dehydrogenase of the present invention to a reaction system under suitable reaction conditions, and performing catalytic conversion on the reaction substrate omeprazole sulfide into esomeprazole.

Specifically, the preparation method of esomeprazole comprises the following steps: first, the genetically engineered bacteria BL21 (DE3)-pETCK-n and phosphate buffer (pH=7.0-10.0) of the present invention were added to the reaction system and stirred evenly, then omeprazole sulfide, isopropanol and coenzyme NADP were sequentially added and reacted under an oxygen atmosphere (0-40°C) for 2-72 h, the omeprazole sulfide content in the liquid phase was controlled ≤ 1 wt%, such as 0.95 wt%, 0.90 wt%, 0.85 wt%, 0.80 wt %, 0.75wt%, 0.70wt%, etc., finally an appropriate amount of ethyl acetate was added to terminate the reaction, the reaction system was stood for layering, the aqueous layer was separated, the reaction product was washed 2-3 times with saturated sodium chloride solution to remove ethyl acetate, then dried and spun, HPLC analysis was performed, and high-purity target product esomeprazole was obtained, with yield ≥ 80%, and ee value ≥98%.

In the above method, the order of addition of the reactants is not critical. The reactants may be added together to the reaction system at the same time; or, optionally, some of the reactants may be added separately and additional reactants may be added at different time points.

According to the process of the invention, the reaction is generally allowed to proceed until the reaction substrate, such as omeprazole sulfide, is substantially completely or nearly completely converted to the target product esomeprazole. Substrate conversion and product formation are detected using known methods, including, but not limited to, gas chromatography, HPLC, and the like.

Compared with the prior art of synthesizing prazole drugs by a biological enzyme method, the invention realizes the high-level co-expression of cyclohexanone monooxygenase and isopropanol dehydrogenase in one strain, changes the fermentation of the cyclohexanone monooxygenase expression strain and the isopropanol dehydrogenase expression strain into the fermentation of one co-expression strain, and reduces the fermentation steps and the production cost; on the other hand, the invention realizes whole cell catalysis, avoids the operations of cell disruption and centrifugation, and has excellent industrial application value.

### Brief Description of the Drawings

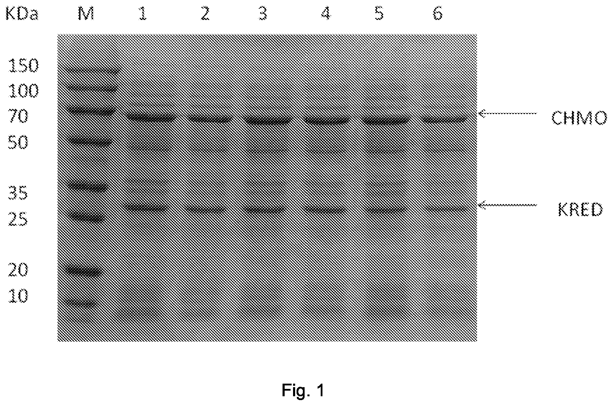

Fig. 1 is an electrophoresis pattern of an induced expression protein of the genetically engineered strain BL21(DE3)-pETCK-n of the present invention. Wherein, M is protein Marker, 1 is the induced bacterial protein of genetic engineering strain BL21(DE3)-pETCK-1, 2 is the induced bacterial protein of genetic engineering strain BL21(DE3)-pETCK-2, 3 is genetically the induced bacterial protein of engineered strain BL21 (DE3)-pETCK-3, 4 is the induced bacterial protein of genetically engineered strain BL21(DE3)-pETCK-4, 5 is the induced bacterial protein of genetically engineered strain BL21(DE3)-pETCK-5, and 6 is the induced bacterial protein of genetically engineered strain BL21 (DE3)-pETCK-6.

### Detailed Description of the Invention

The present invention will be further described in detail below in conjunction with specific embodiments. It is understood that the following examples are merely illustrative of the invention and are not intended to limit the scope of the invention.

The amounts, contents, concentrations, and percentages of the various materials referred to herein are by mass unless otherwise indicated.

In this context, sometimes for the sake of simplicity of description, the name of a certain protein is used in combination with the name of the encoding gene (DNA), and those skilled in the art will understand that they represent different substances in different descriptions. For example, for a cyclohexanone monooxygenase (gene), when used to describe its catalytic substrate reaction, it refers to a protein; when described as a gene, it refers to the gene encoding the cyclohexanone monooxygenase, and so on.

The microorganism co-expressing cyclohexanone monooxygenase and isopropanol dehydrogenase may be any transformant host, including, for example, but not limited to, bacteria and fungi. Preferably bacteria, especially *Escherichia coli,* including, for example, but not limited to *Escherichia coli* BL21 (DE3), *Escherichia coli* BL21 (DE3) plysS or *Escherichia coli* BL21 (DE3) star.

When used as a bio-enzyme catalyst for the production of esomeprazole, any form of co-expression of cyclohexanone monooxygenase and isopropanol dehydrogenase may be used in the present invention, for example, in the form of an enzyme, a microorganism, or other forms. The forms of enzymes comprise free enzyme and immobilized enzyme, including purified enzyme, crude enzyme, fermentation liquor, carrier-immobilized enzyme and the like; the bacterial forms include viable and dead bacteria. The form of genetically engineered strain is preferred.

The primers T7 and T7term used in the invention are all conventional primers and can be synthesized by the skilled in the art according to the prior art or obtained commercially available. Plasmids used in the present invention, such as Plasmid pET 21a, Plasmid pET 28a, Plasmid pET 22b, Plasmid pETDuet1, Plasmid pRSFDuet 1, or Plasmid pACYCDuet 1, are all conventional in the art and are commercially available.

The LB medium and the fermentation medium of the present invention are conventional products, and can be prepared by those skilled in the art according to the prior art or commercially available, for example, they can be prepared by themselves with reference to Molecular Cloning: A Laboratory Manual (Third Edition, J. Sambrook, D.W. Russell (United States), Huang Peitang et al., Science Press, Beijing, 2002). For example, the LB medium can be prepared in the following formula: yeast extract 5 g, peptone 10 g, NaCl 5 g, agar 15-20 g, adding water to 1000 mL, and pH=7.4-7.6. The term "LB medium with corresponding resistance" means that an appropriate amount of an antibiotic, which is slightly different depending on the plasmid selected but is conventionally selected in the art, such as ampicillin and/or kanamycin, is added to the LB medium when it is prepared. The fermentation medium can be prepared by the following formula: Na₂HPO₄ 6g, KH₂PO₄ 3g, NaCl 0.5g, NH₄Cl 1g, MgSO₄•7H₂O 0.5g, CaCl₂ 0.011g, glucose 2-10g, adding water to 1000 mL, and pH=7.0.

### Example 1 Synthesis and site-directed mutagenesis of a cyclohexanone monooxygenase gene fragment

The gene fragment was synthesized by querying cyclohexanone-1,2-monooxygenase gene 1 (NCBI accession number 578026767) through NCBI, wherein the nucleotide sequence of the gene fragment is shown in SEQ ID NO. 1, and the corresponding encoded amino acid sequence is shown in SEQ ID NO: 2.

The above cyclohexanone monooxygenase gene was protein engineered using the QuickChange Site-directed Mutagenesis (Stratagene) method (Agilent Technologies). Specifically, the following primers F1 and R1 were designed (the underlined positions correspond to the corresponding mutation sites), and the serine (Ser) at the 435th position of the amino acid sequence SEQ ID NO: 2 of the cyclohexanone monooxygenase gene was subjected to site-directed mutagenesis to be mutated into threonine (Thr):
Forward primer F1: GGTCCACTGGCCAAT ACT CCTCCTATCATCG (SEQ ID NO. 25)
Reverse primer R1: CGATGATAGGAGG AGT ATTGGCCAGTGGACC (SEQ ID NO. 26)
PCR setup:
   pre-denaturation at 95°C for 3 min;
   95°C, 45 s; 55°C, 45 s; 72°C, 2 min, 18 cycles;
   72°C, 7 min.

The cyclohexanone monooxygenase mutant gene 2 of the present invention was obtained, the nucleotide sequence of which is shown in SEQ ID NO: 3, and the corresponding amino acid sequence is shown in SEQ ID NO: 4.

The above cyclohexanone monooxygenase gene 2 was protein engineered using the QuickChange Site-directed Mutagenesis (Stratagene) method (Agilent Technologies). Specifically, the following primers F2 and R3 were designed (the underlined positions correspond to the corresponding mutation sites), and the serine (Ser) at the 386th position of the amino acid sequence SEQ ID NO: 3 of the cyclohexanone monooxygenase gene was subjected to site-directed mutagenesis to be mutated into asparagine (Asn):
Forward primer F2: GCTTTGATGCGGGCGATGGC AAC TACAAGCGCATCGACATACAG (SEQ ID NO. 27)
Reverse Primer R2: GTT GCCATCGCCCGCATCAAAGCCGGTCGCGCAAATC (SEQ ID NO. 28)
PCR setup:
   pre-denaturation at 95°C for 3 min;
   95°C, 45 s; 55°C, 45 s; 72°C, 2 min, 18 cycles;
   72°C, 7 min.

The cyclohexanone monooxygenase mutant gene 3 of the present invention was obtained, the nucleotide sequence of which is shown in SEQ ID NO: 5, and the corresponding amino acid sequence is shown in SEQ ID NO: 6.

Similarly, the gene fragment (adding NcoI and BamH I endonuclease fragments to both ends of the fragment) was synthesized by querying cyclohexanone-1,2-monooxygenase gene 4 (NCBI accession number 296848397) through NCBI, wherein the nucleotide sequence is shown in SEQ ID NO. 7, and the corresponding encoded amino acid sequence is shown in SEQ ID NO. 8.

The gene fragment (adding NcoI and Hind III endonuclease fragments to both ends of the fragment) was synthesized by querying cyclohexanone-1,2-monooxygenase gene 5 (NCBI accession number 1109637906) through NCBI, wherein the nucleoside acid sequence is shown in SEQ ID NO. 9, and the corresponding encoded amino acid sequence is shown in SEQ ID NO.10.

The gene fragment was synthesized by querying cyclohexanone-1,2-monooxygenase gene 6 (NCBI accession number 1099765055) through NCBI, wherein the nucleotide sequence is shown in SEQ ID NO. 11, and the corresponding encoded amino acid sequence is shown in SEQ ID NO. 12.

### Example 2 Synthesis of isopropanol dehydrogenase gene fragment

The gene fragment was synthesized by querying isopropanol dehydrogenase gene 1 (NCBI accession number 578026822) through NCBI, wherein the nucleotide sequence is shown in SEQ ID NO. 13, and the corresponding encoded amino acid sequence is shown in SEQ ID NO. 14.

The gene fragment was synthesized by querying isopropanol dehydrogenase gene 2 (NCBI accession number 938992178) through NCBI, wherein the nucleotide sequence is shown in SEQ ID NO. 15, and the corresponding encoded amino acid sequence is shown in SEQ ID NO. 16.

The gene fragment was synthesized by querying isopropanol dehydrogenase gene 3 (NCBI accession number 931139619) through NCBI, wherein the nucleotide sequence is shown in SEQ ID NO. 17, and the corresponding encoded amino acid sequence is shown in SEQ ID NO.18.

The gene fragment (adding NdeI and XhoI endonuclease fragments to both ends of the fragment) was synthesized by querying isopropanol dehydrogenase gene 4 (NCBI accession number 21225427) through NCBI, wherein the nucleotide sequence thereof is shown in SEQ ID NO. 19, and the corresponding encoded amino acid sequence is shown in SEQ ID NO.20.

The gene fragment (adding NdeI and KpnI endonuclease fragments to both ends of the fragment) was synthesized by querying isopropanol dehydrogenase gene 5 (NCBI accession number 874510474) through NCBI, wherein the nucleotide sequence is shown in SEQ ID NO. 21, and the corresponding encoded amino acid sequence is shown in SEQ ID NO.22.

The gene fragment (adding Nde I and Xho I endonuclease fragments to both ends of the fragment) was synthesized by querying isopropanol dehydrogenase gene 6 (NCBI accession number 1089568368) through NCBI, wherein the nucleotide sequence thereof is shown in SEQ ID NO. 23, and the corresponding encoded amino acid sequence is shown in SEQ ID NO.24.

### Example 3 Construction of recombinant co-expression vector

The following experiments such as enzyme digestion and ligation or preparation or transformation of competent cells were carried out with reference to Molecular Cloning: A Laboratory Manual (Third Edition, J. Sambrook, D.W. Russell (United States), Huang Peitang et al., Science Press, Beijing, 2002).
Forward primer F3: GGCCATATGAATAATTTTGTTTAACTTTAAGAAGGAGATATAA TGAGTACCAAGATGGATTTTG (SEQ ID NO. 29)
Reverse primer R3: CGCGGATCCTTACGCATTAGCCTGCTGTTTGG (SEQ ID NO. 30)

Primers F3 and R3 were designed, and the three cyclohexanone monooxygenase gene fragments with the nucleotide sequences of SEQ ID NO. 1, SEQ ID NO. 3 and SEQ ID NO. 5 were respectively used as templates, and the cyclohexanone monooxygenase gene fragments (adding Nde I and BamH I endonuclease fragments to both ends of the fragment) were expanded by PCR amplification respectively; the three genes were inserted into Plasmid pET 21a, Plasmid pET 28a or Plasmid pET 22b by Nde I and BamH I endonuclease sites, and the vector obtained after ligation was transferred into *E. coli* Trans-Tl competent cells to construct the recombinant plasmid which was verified by colony PCR with primer T7/R3, then was extracted and verified to obtain the correct recombinant plasmid, which was named as pETC-n.

Forward primer F4:

CGCGGATCCAAGCTTAATAATTTTGTTTAACTTTAAGAAGGAGATATAATGACGGATCGT
CTGAAAGGC (SEQ ID NO. 31)

Reverse primer R4: CCGCTCGAGTTACTGGGCGGTATAGCCGCC (SEQ ID NO. 32)

Primers F4 and R4 were designed, and the three isopropanol dehydrogenase gene fragments with the nucleotide sequences of SEQ ID NO. 13, SEQ ID NO. 15 and SEQ ID NO. 17 were respectively used as templates, and the isopropanol dehydrogenase gene fragments (adding BamH I and Xho I endonuclease fragments to both ends of the fragment) were expanded by PCR amplification; the three genes were inserted into the recombinant plasmid pETC-n by BamH I and Xho I endonuclease sites, and the vector obtained after ligation was transferred into *E. coli* Trans-Tl competent cells to construct the recombinant plasmid which was verified by colony PCR with primer F4/T7term, then was extracted and verified by sequencing to obtain the correct recombinant co-expression vector, which was named as pETCK-n.

Similarly, by using the same method, the three cyclohexanone monooxygenase gene fragments with the nucleotide sequences of SEQ ID NO. 7, SEQ ID NO. 9 and SEQ ID NO. 11 were respectively used as templates, and the recombinant plasmid pETC-m was obtained by restriction enzyme insertion using Plasmid pETDuet 1, Plasmid pRSFDuet1 and Plasmid pACYCDuet 1.

Then, the three isopropanol dehydrogenase gene fragments with the nucleotide sequences of SEQ ID NO. 19, SEQ ID NO. 21 and SEQ ID NO. 23 were respectively used as template, and the recombinant co-expression vector pETCK-m was constructed according to the same method as above.

The information of each recombinant expression vector is as described in Table 1 below.

**Table 1 Construction information of each recombinant co-expression vector**

| Recombinant co-expression vector number | plasmid | nucleotide sequence | |
|---|---|---|---|
| | | cyclohexanone monooxygenase | isopropanol dehydrogenase |
| pETCK-1 | pET 21a | SEQ ID NO.1 | SEQ ID NO.15 |
| pETCK-2 | pET 28a | SEQ ID NO.3 | SEQ ID NO.13 |
| pETCK-3 | pET 22b | SEQ ID NO.5 | SEQ ID N0.17 |
| pETCK-4 | pETDuet | SEQ ID NO.7 | SEQ ID NO.19 |
| pETCK-5 | pRSFDuet | SEQ ID NO.9 | SEQ ID NO.21 |
| pETCK-6 | pACYCDuet | SEQ ID NO.11 | SEQ ID NO.23 |

### Example 4: Construction of genetically engineered strain

Each recombinant co-expression vector constructed in the above Example 3 was transformed into competent cells *Escherichia coli* BL21 (DE3), *Escherichia coli* BL21 (DE3) plysS or *Escherichia coli* BL21 (DE3) star (provided by TransGen Biotech Co., Ltd.) respectively. Colony PCR was performed with primer T7/T7term. After verification, the genetically engineered strain co-expressing cyclohexanone monooxygenase and isopropanol dehydrogenase in the present invention was obtained, and named as BL21(DE3)-pETCK-n. The construction information is shown in Table 2 below.

**Table 2 Construction information of genetically engineered strains BL21 (DE3)-pETCK-n**

| Genetically engineered strain number | Host bacteria | Recombinant co-expression vector number |
|---|---|---|
| BL21 (DE3)-pETCK-1 | *Escherichia coli* BL21(DE3) plysS | pETCK-1 |
| BL21 (DE3)-pETCK-2 | *Escherichia coli* BL21(DE3) | pETCK-2 |
| BL21 (DE3)-pETCK-3 | *Escherichia coli* BL21(DE3) star | pETCK-3 |
| BL21 (DE3)-pETCK-4 | *Escherichia coli* BL21(DE3) | pETCK-4 |
| BL21 (DE3)-pETCK-5 | *Escherichia coli* BL21(DE3) | pETCK-5 |
| BL21 (DE3)-pETCK-6 | *Escherichia coli* BL21(DE3) | pETCK-6 |

### Example 5 Induced expression of genetically engineered strain by shaking culture

The genetically engineered strain BL21(DE3)-pETCK-n obtained by the above construction was inoculated into a 3-5 mL of liquid LB test tube culture medium containing corresponding resistance, and activated by shaking at 37°C for 12-36 h; the activated culture was transferred to the fermentation medium at an inoculation amount of 0.5%-5% (v/v), fermented at 25-40°C for 1-3 h, and then added with an inducer isopropyl-beta-D-thiogalactopyranoside (IPTG) with the final concentration of 0.05-2 g/L, and induced to express at 15-35°C for 2-24h; and the bacterial sludge is obtained through centrifugation, filtration or ultrafiltration of the expression product, and cryopreserved at -80°C. The culture conditions are shown in Table 3.

**Table 3 Expression conditions of shaking culture of genetically engineered strains**

| Genetically engineered strain number | Inoculation amount (%) | Culture temperature (°C) | Cultivation time (h) | Inducer concentration (g/L) | Induction temperature (°C) | Induction time (h) |
|---|---|---|---|---|---|---|
| BL21(DE3)-pETCK-1 | 0.5 | 25 | 1 | 2 | 15 | 24 |
| BL21(DE3)-pETCK-2 | 1 | 37 | 2 | 1.5 | 20 | 16 |
| BL21(DE3)-pETCK-3 | 2 | 40 | 3 | 1.2 | 28 | 12 |
| BL21(DE3)-pETCK-4 | 3 | 37 | 3 | 1 | 30 | 8 |
| BL21(DE3)-pETCK-5 | 4 | 37 | 3 | 0.08 | 35 | 5 |
| BL21(DE3)-pETCK-6 | 5 | 37 | 3 | 0.05 | 37 | 4 |

Figure 1 shows the protein expression of each genetically engineered strain BL21 (DE3)-pETCK-n detected by SDS-PAGE.

### Example 6 Enzyme Activity Assay

The enzyme activity of the genetically engineered strain BL21(DE3)-pETCK-n of the present invention was determined by the following method:
2 mL of the fermentation broth of the genetically engineered strain obtained in Example 5 was taken respectively, the bacteria was collected by centrifuging, and the supernatant was poured out; 1 mL potassium phosphate buffer (50 mmol, pH=8.0), 0.2 g/L oxidized coenzyme II, 10 mmol/L omeprazole sulfide and 10% isopropanol were added into the bacteria, and vortex mixing was performed; the mixture was shaken in a shaker (200 rpm) at 37°C for 1 h, the amount of esomeprazole by HPLC was detected, and the enzyme activity was calculated.

The enzyme activity per unit is defined as the amount of enzyme required to catalyze the production of 1 µmol of esomeprazole per hour.

HPLC detection method is as follows: Shimadzu liquid chromatography, Phenomenex chromatographic column, 10 mM phosphate (pH=7.6): acetonitrile (60: 40) as mobile phase, flow rate of 1 mL/min, column temperature of 35°C, UV detection wavelength of 300nm, the detection time of 15min, and retention time: product (3.8 min), substrate (8.2 min).

The results of the enzyme activity are shown in Table 4. It can be seen from Table 4 that the genetically engineered strain BL21 (DE3)-pETCK-2 has the best enzyme activity, followed by the genetically engineered strain BL21(DE3)-pETCK-3, both of which are higher than that of genetically engineered strain BL21 (DE3)-pETCK-1; the applicant speculated that the reason may be due to the construction of the first two genetically engineered strain based on the mutation of the cyclohexanone-1,2-monooxygenase gene; the enzyme activities of genetically engineered strain BL21 (DE3)-pETCK-4, BL21(DE3)-pETCK-5, and BL21(DE3)-pETCK-6 were relatively poor.

**Table 4: Enzyme activity of the genetically engineered strains of the present invention**

| Genetically engineered strain number | Enzyme activity (µmol/g^{∗}h) |
|---|---|
| BL21 (DE3)-pETCK-1 | 86.1 |
| BL21 (DE3)-pETCK-2 | 112.4 |
| BL21 (DE3)-pETCK-3 | 96.1 |
| BL21 (DE3)-pETCK-4 | 18.4 |
| BL21 (DE3)-pETCK-5 | 7.6 |
| BL21 (DE3)-pETCK-6 | 14.2 |

### Example 7 Enzyme-catalyzed preparation of esomeprazole

10 g of the fermentation broth of the genetically engineered strain BL21(DE3)-pETCK-1, BL21(DE3)-pETCK-2, and BL21(DE3)-pETCK-3 obtained in Example 5 were weighed respectively, and 80 mL of phosphate buffer (pH=7.0-10.0) was added to stir evenly, 1 g of omeprazole sulfide, 20 mL of isopropanol, and 0.006 mg of oxidized coenzyme II were added in proper order, temperature (0-40°C) was kept under oxygen environment for reaction 2-72h, the content of omeprazole sulfide in the liquid phase was controlled <1wt%, 200mL of ethyl acetate was added to terminate the reaction, the reaction system was stood for layering, the aqueous layer was separated, the reaction product was washed 2 times with saturated sodium chloride solution to remove ethyl acetate, then dried and spun, HPLC analysis was performed, and high-purity target product esomeprazole was obtained, with ee value>98% , and yield>80%.

The reaction conditions of each genetically engineered strain are shown in Table 5.

**Table 5: Reaction conditions and results of genetically engineered strains**

| Genetically engineered strain number | pH | Temperature (°C) | Reaction time (h) | ee value (%) |
|---|---|---|---|---|
| BL21(DE3)-pETCK-1 | 7 | 40 | 2 | 98.6 |
| BL21(DE3)-pETCK-2 | 9 | 30 | 18 | 99.1 |
| BL21(DE3)-pETCK-3 | 10 | 0 | 72 | 99.5 |

The above examples are only for illustrating the technical solution of the present invention, and are not intended to limit the present invention. Although the present invention has been described in detail with reference to the foregoing embodiments, it should be understood by those skilled in the art that modifications to the technical solutions described in the foregoing embodiments or equivalent substitutions of some of the technical features may be made without departing from the spirit and scope of the technical solutions of the present invention.

## Claims

1. A genetically engineered strain for co-expressing cyclohexanone monooxygenase and isopropanol dehydrogenase.

2. The genetically engineered strain according to claim 1, **characterized in that** the genetically engineered strain comprises a cyclohexanone monooxygenase gene and an isopropanol dehydrogenase gene simultaneously.

3. The genetically engineered strain according to claim 1 or 2, **characterized in that** the genetically engineered strain is constructed as follows:
(1) using the cyclohexanone monooxygenase gene fragment as a template, performing PCR amplification, inserting the gene fragment into plasmids using restriction endonuclease Nde I and BamH I recognition sites, and transferring the vector obtained after ligation into *E. coli* Trans-Tl competent cells to construct a recombinant plasmid pETC-n;
(2) using the isopropanol dehydrogenase gene fragment as a template, performing PCR amplification, inserting the gene fragment into the recombinant plasmid pETC-n obtained by above-mentioned step (1) using restriction endonuclease BamH I and Xho I recognition sites, and transferring the vector obtained after ligation into *E. coli* Trans-Tl competent cells to construct a recombinant expression vector pETCK-n;
(3) transforming the recombinant expression vector pETCK-n obtained by the above construction into an expression host, performing colony PCR with primer T7/T7 term, after verification, and obtaining genetically engineered strain BL21(DE3)-pETCK-n for co-expressing cyclohexanone monooxygenase gene and isopropanol dehydrogenase gene.

4. The genetically engineered strain according to claim 3, **characterized in that** the plasmids in the step (1) are selected from the group consisting of Plasmid pET 21a, Plasmid pET 28a, Plasmid pET 22b, Plasmid pETDuet 1, Plasmid pRSFDuet 1, or Plasmid pACYCDuet 1.

5. The genetically engineered strain according to claim 3, **characterized in that** the forward and reverse primers sequences of the PCR amplification designed in step (1) are respectively shown in SEQ ID NO. 29 and SEQ ID NO. 30; and the forward and reverse primers sequences of the PCR amplification designed in step (2) are shown in SEQ ID NO. 31 and SEQ ID NO. 32.

6. The genetically engineered strain according to claim 3, **characterized in that** the host in the step (3) is *Escherichia coli* BL21 (DE3), *Escherichia coli* BL21 (DE3) plysS or *Escherichia coli* BL21 (DE3) star.

7. The genetically engineered strain according to claim 1, 2 or 3, **characterized in that** the amino acid sequence of cyclohexanone monooxygenase is selected from the group consisting of SEQ ID NO. 2, SEQ ID NO. 4, SEQ ID NO. 6. SEQ ID NO. 8, SEQ ID NO. 10, and SEQ ID NO. 12; and the amino acid sequence of isopropanol dehydrogenase is selected from the group consisting of SEQ ID NO. 14, SEQ ID NO. 16, SEQ ID NO. 18. SEQ ID NO. 20, SEQ ID NO. 22, and SEQ ID NO. 24.

8. The genetically engineered strain according to claim 7, **characterized in that** the nucleotide sequence encoding the cyclohexanone monooxygenase gene is correspondingly selected from the group consisting of SEQ ID NO. 1, SEQ ID NO. 3, SEQ ID NO. 5, SEQ ID NO. 7, SEQ ID NO. 9, and SEQ ID NO. 11 respectively; and the nucleotide sequence encoding the isopropanol dehydrogenase gene is correspondingly selected from the group consisting of SEQ ID NO. 13, SEQ ID NO. 15, SEQ ID NO. 17, SEQ ID NO. 19, SEQ ID NO. 21, and SEQ ID NO. 23 respectively.

9. A method for preparing esomeprazole, **characterized in that** the genetically engineered strain according to any one of claims 1 to 8 is added to a reaction system, and catalytic conversion is performed on the reaction substrate omeprazole sulfide into esomeprazole under suitable reaction conditions.

10. The method according to claim 9, **characterized in that** the content of omeprazole sulfide in the liquid phase of the reaction system is controlled to be ≤ 1 wt%.


SEQUENCE LISTING

<110>  ZHEJIANG JINGXIN PHARMACEUTICAL CO., LTD.
       SHANGHAI JINGXIN BIOLOGY & PHARMACEUTICAL CO., LTD.

<120>  GENETICALLY ENGINEERED BACTERIUM OF COEXPRESSING CYCLOHEXANONE
MONOOXYGENASE AND ISOPROPANOL DEHYDROGENASE AND USE THEREOF

<130>  001417P-WOEP

<140>  EP17883408.1

<141>  23-05-2019

<160>  32

<170>  PatentIn version 3.5

<210>  1
<211>  1632
<212>  DNA
<213>  Artificial synthesis

<400>  1
atgagtacca agatggattt tgatgcaatt gtgatcggtg ccggctttgg cggcctgtat      60
gccgttaaaa aactgcgcga tgaactggaa ctgaaagtta aagcctttga taaagcaacg     120
gatgtgggcg ggacctggta ttggaatcgc tatccgggcg cactgacgga tacggaaacc     180
catctgtatt gctattcttg ggataaagaa atgctacaga gtttagaaat caaaaagaaa     240
tatgtgcagg gcccagatgt tcgcaaatac ttacagcagg ttgcagaaaa acatgatctg     300
aaaaaatctt atcagtttaa taccgccgtg acgagtgctc attataacga ggcggatgcc     360
ctgtgggaag tgacaaccga atatggcgat aaatataccg cacgctttct gattaccgcc     420
gtgggtctgc tgtctgcacc taattggcct aatatcaaag gcatcaatca gtttaaaggt     480
gaactgcatc atacgtcacg ctggccggat gatgtgagca tcgaaggcaa gagagtgggt     540
gtgatcggta cgggtagtac gggcgttcag gttattacag cagttgctcc attagccaaa     600
catctgaccg tgtttcagcg tagtccacag tatagtgttc cgatcggcaa tgatccactg     660
agcgaagaag atgttaagaa gattaaagat aattatgata aaatctggga tggtgtgaaa     720
aatagcgcct tagcctatgg tgtgaatgag tctacagttc cagccatgag cgtgagtgca     780
gaagaacgta aagccgtgtt tgaaaaggca tggcagacgg gtggcgggat gcgctttatg     840
tttgaaacct ttggggacat aattaccaat atggaagcta atatcgaagc acagaatttt     900
atcaaaggca aaattgcccg catcgttaaa gatcctgcca ttgcacagaa actgatgcct     960
caggatctgt atgcttgtcg cccgctgtgc gattcaggct attataatac ctttaatcgc    1020
gaaaatgttc gtctggaaga tgttaaagct aatccgatcg tggaaatcac cgaaaatggc    1080
gttaaactgg aaaatggcga ttttgtggaa ttagatatgc tgatttgcgc gaccggcttt    1140
gatgcgggcg atggctctta caagcgcatc gacatacagg ggaaaaatgg cttagccatc    1200
aaagattatt ggaaagaagg tcctagtagc tatatgggcg ttgcggttaa caactatccg    1260
aatatgttta tggtttttgg tccgaatggt ccactggcca attctcctcc tatcatcgaa    1320
tctcaggttg agtggatttc agtgtttatc cagtataccg ttgaaaacaa tgttgaatct    1380
atcgaagccg ataaagaagc ggaagaacag tggacccaga cctgcgccaa tattgccgaa    1440
aaaaccctgt ttcctaaagc caaatgtcgc atctttggtg ccaatattcc gggcaagaaa    1500
aacacggtgt atctgtacct cggcggtctg aaagaatatc gtagcgtttt agctaattgc    1560
aaaaatcatg cgtatgtggg ctttgatatt cagttacagc gctcagatac caaacagcag    1620
gctaatgcgt aa                                                        1632

<210>  2
<211>  543
<212>  PRT
<213>  Artificial synthesis

<400>  2
Met Ser Thr Lys Met Asp Phe Asp Ala Ile Val Ile Gly Ala Gly Phe
1               5                   10                  15
Gly Gly Leu Tyr Ala Val Lys Lys Leu Arg Asp Glu Leu Glu Leu Lys
            20                  25                  30

Val Lys Ala Phe Asp Lys Ala Thr Asp Val Gly Gly Thr Trp Tyr Trp
            35                  40                  45
Asn Arg Tyr Pro Gly Ala Leu Thr Asp Thr Glu Thr His Leu Tyr Cys
    50                  55                  60
Tyr Ser Trp Asp Lys Glu Met Leu Gln Ser Leu Glu Ile Lys Lys Lys
65                  70                  75                  80
Tyr Val Gln Gly Pro Asp Val Arg Lys Tyr Leu Gln Gln Val Ala Glu
                85                  90                  95
Lys His Asp Leu Lys Lys Ser Tyr Gln Phe Asn Thr Ala Val Thr Ser
            100                 105                 110
Ala His Tyr Asn Glu Ala Asp Ala Leu Trp Glu Val Thr Thr Glu Tyr
        115                 120                 125
Gly Asp Lys Tyr Thr Ala Arg Phe Leu Ile Thr Ala Val Gly Leu Leu
    130                 135                 140
Ser Ala Pro Asn Trp Pro Asn Ile Lys Gly Ile Asn Gln Phe Lys Gly
145                 150                 155                 160
Glu Leu His His Thr Ser Arg Trp Pro Asp Asp Val Ser Ile Glu Gly
                165                 170                 175
Lys Arg Val Gly Val Ile Gly Thr Gly Ser Thr Gly Val Gln Val Ile
            180                 185                 190
Thr Ala Val Ala Pro Leu Ala Lys His Leu Thr Val Phe Gln Arg Ser
        195                 200                 205
Pro Gln Tyr Ser Val Pro Ile Gly Asn Asp Pro Leu Ser Glu Glu Asp
    210                 215                 220
Val Lys Lys Ile Lys Asp Asn Tyr Asp Lys Ile Trp Asp Gly Val Lys
225                 230                 235                 240
Asn Ser Ala Leu Ala Tyr Gly Val Asn Glu Ser Thr Val Pro Ala Met
                245                 250                 255
Ser Val Ser Ala Glu Glu Arg Lys Ala Val Phe Glu Lys Ala Trp Gln
            260                 265                 270
Thr Gly Gly Gly Met Arg Phe Met Phe Glu Thr Phe Gly Asp Ile Ile
        275                 280                 285
Thr Asn Met Glu Ala Asn Ile Glu Ala Gln Asn Phe Ile Lys Gly Lys
    290                 295                 300
Ile Ala Arg Ile Val Lys Asp Pro Ala Ile Ala Gln Lys Leu Met Pro
305                 310                 315                 320
Gln Asp Leu Tyr Ala Cys Arg Pro Leu Cys Asp Ser Gly Tyr Tyr Asn
                325                 330                 335
Thr Phe Asn Arg Glu Asn Val Arg Leu Glu Asp Val Lys Ala Asn Pro
            340                 345                 350
Ile Val Glu Ile Thr Glu Asn Gly Val Lys Leu Glu Asn Gly Asp Phe
        355                 360                 365
Val Glu Leu Asp Met Leu Ile Cys Ala Thr Gly Phe Asp Ala Gly Asp
    370                 375                 380
Gly Ser Tyr Lys Arg Ile Asp Ile Gln Gly Lys Asn Gly Leu Ala Ile
385                 390                 395                 400
Lys Asp Tyr Trp Lys Glu Gly Pro Ser Ser Tyr Met Gly Val Ala Val
                405                 410                 415
Asn Asn Tyr Pro Asn Met Phe Met Val Phe Gly Pro Asn Gly Pro Leu
            420                 425                 430
Ala Asn Ser Pro Pro Ile Ile Glu Ser Gln Val Glu Trp Ile Ser Val
        435                 440                 445
Phe Ile Gln Tyr Thr Val Glu Asn Asn Val Glu Ser Ile Glu Ala Asp
    450                 455                 460
Lys Glu Ala Glu Glu Gln Trp Thr Gln Thr Cys Ala Asn Ile Ala Glu
465                 470                 475                 480
Lys Thr Leu Phe Pro Lys Ala Lys Cys Arg Ile Phe Gly Ala Asn Ile
                485                 490                 495
Pro Gly Lys Lys Asn Thr Val Tyr Leu Tyr Leu Gly Gly Leu Lys Glu
            500                 505                 510
Tyr Arg Ser Val Leu Ala Asn Cys Lys Asn His Ala Tyr Val Gly Phe
        515                 520                 525
Asp Ile Gln Leu Gln Arg Ser Asp Thr Lys Gln Gln Ala Asn Ala

530                 535                 540

<210>  3
<211>  1632
<212>  DNA
<213>  Artificial synthesis

<400>  3
atgagtacca agatggattt tgatgcaatt gtgatcggtg ccggctttgg cggcctgtat      60
gccgttaaaa aactgcgcga tgaactggaa ctgaaagtta aagcctttga taaagcaacg     120
gatgtgggcg ggacctggta ttggaatcgc tatccgggcg cactgacgga tacggaaacc     180
catctgtatt gctattcttg ggataaagaa atgctacaga gtttagaaat caaaaagaaa     240
tatgtgcagg gcccagatgt tcgcaaatac ttacagcagg ttgcagaaaa acatgatctg     300
aaaaaatctt atcagtttaa taccgccgtg acgagtgctc attataacga ggcggatgcc     360
ctgtgggaag tgacaaccga atatggcgat aaatataccg cacgctttct gattaccgcc     420
gtgggtctgc tgtctgcacc taattggcct aatatcaaag gcatcaatca gtttaaaggt     480
gaactgcatc atacgtcacg ctggccggat gatgtgagca tcgaaggcaa gagagtgggt     540
gtgatcggta cgggtagtac gggcgttcag gttattacag cagttgctcc attagccaaa     600
catctgaccg tgtttcagcg tagtccacag tatagtgttc cgatcggcaa tgatccactg     660
agcgaagaag atgttaagaa gattaaagat aattatgata aaatctggga tggtgtgaaa     720
aatagcgcct tagcctatgg tgtgaatgag tctacagttc cagccatgag cgtgagtgca     780
gaagaacgta aagccgtgtt tgaaaaggca tggcagacgg gtggcgggat gcgctttatg     840
tttgaaacct ttggggacat aattaccaat atggaagcta atatcgaagc acagaatttt     900
atcaaaggca aaattgcccg catcgttaaa gatcctgcca ttgcacagaa actgatgcct     960
caggatctgt atgcttgtcg cccgctgtgc gattcaggct attataatac ctttaatcgc    1020
gaaaatgttc gtctggaaga tgttaaagct aatccgatcg tggaaatcac cgaaaatggc    1080
gttaaactgg aaaatggcga ttttgtggaa ttagatatgc tgatttgcgc gaccggcttt    1140
gatgcgggcg atggctctta caagcgcatc gacatacagg ggaaaaatgg cttagccatc    1200
aaagattatt ggaaagaagg tcctagtagc tatatgggcg ttgcggttaa caactatccg    1260
aatatgttta tggtttttgg tccgaatggt ccactggcca atactcctcc tatcatcgaa    1320
tctcaggttg agtggatttc agtgtttatc cagtataccg ttgaaaacaa tgttgaatct    1380
atcgaagccg ataaagaagc ggaagaacag tggacccaga cctgcgccaa tattgccgaa    1440
aaaaccctgt ttcctaaagc caaatgtcgc atctttggtg ccaatattcc gggcaagaaa    1500
aacacggtgt atctgtacct cggcggtctg aaagaatatc gtagcgtttt agctaattgc    1560
aaaaatcatg cgtatgtggg ctttgatatt cagttacagc gctcagatac caaacagcag    1620
gctaatgcgt aa                                                         1632

<210>  4
<211>  543
<212>  PRT
<213>  Artificial synthesis

<400>  4
Met Ser Thr Lys Met Asp Phe Asp Ala Ile Val Ile Gly Ala Gly Phe
1               5                   10                  15
Gly Gly Leu Tyr Ala Val Lys Lys Leu Arg Asp Glu Leu Glu Leu Lys
            20                  25                  30
Val Lys Ala Phe Asp Lys Ala Thr Asp Val Gly Gly Thr Trp Tyr Trp
        35                  40                  45
Asn Arg Tyr Pro Gly Ala Leu Thr Asp Thr Glu Thr His Leu Tyr Cys
    50                  55                  60
Tyr Ser Trp Asp Lys Glu Met Leu Gln Ser Leu Glu Ile Lys Lys Lys
65                  70                  75                  80
Tyr Val Gln Gly Pro Asp Val Arg Lys Tyr Leu Gln Gln Val Ala Glu
                85                  90                  95
Lys His Asp Leu Lys Lys Ser Tyr Gln Phe Asn Thr Ala Val Thr Ser
            100                 105                 110
Ala His Tyr Asn Glu Ala Asp Ala Leu Trp Glu Val Thr Thr Glu Tyr
        115                 120                 125
Gly Asp Lys Tyr Thr Ala Arg Phe Leu Ile Thr Ala Val Gly Leu Leu
    130                 135                 140
Ser Ala Pro Asn Trp Pro Asn Ile Lys Gly Ile Asn Gln Phe Lys Gly
145                 150                 155                 160

Glu Leu His His Thr Ser Arg Trp Pro Asp Asp Val Ser Ile Glu Gly
                165                 170                 175
Lys Arg Val Gly Val Ile Gly Thr Gly Ser Thr Gly Val Gln Val Ile
            180                 185                 190
Thr Ala Val Ala Pro Leu Ala Lys His Leu Thr Val Phe Gln Arg Ser
        195                 200                 205
Pro Gln Tyr Ser Val Pro Ile Gly Asn Asp Pro Leu Ser Glu Glu Asp
    210                 215                 220
Val Lys Lys Ile Lys Asp Asn Tyr Asp Lys Ile Trp Asp Gly Val Lys
225                 230                 235                 240
Asn Ser Ala Leu Ala Tyr Gly Val Asn Glu Ser Thr Val Pro Ala Met
                245                 250                 255
Ser Val Ser Ala Glu Glu Arg Lys Ala Val Phe Glu Lys Ala Trp Gln
            260                 265                 270
Thr Gly Gly Gly Met Arg Phe Met Phe Glu Thr Phe Gly Asp Ile Ile
        275                 280                 285
Thr Asn Met Glu Ala Asn Ile Glu Ala Gln Asn Phe Ile Lys Gly Lys
    290                 295                 300
Ile Ala Arg Ile Val Lys Asp Pro Ala Ile Ala Gln Lys Leu Met Pro
305                 310                 315                 320
Gln Asp Leu Tyr Ala Cys Arg Pro Leu Cys Asp Ser Gly Tyr Tyr Asn
                325                 330                 335
Thr Phe Asn Arg Glu Asn Val Arg Leu Glu Asp Val Lys Ala Asn Pro
            340                 345                 350
Ile Val Glu Ile Thr Glu Asn Gly Val Lys Leu Glu Asn Gly Asp Phe
        355                 360                 365
Val Glu Leu Asp Met Leu Ile Cys Ala Thr Gly Phe Asp Ala Gly Asp
    370                 375                 380
Gly Ser Tyr Lys Arg Ile Asp Ile Gln Gly Lys Asn Gly Leu Ala Ile
385                 390                 395                 400
Lys Asp Tyr Trp Lys Glu Gly Pro Ser Ser Tyr Met Gly Val Ala Val
                405                 410                 415
Asn Asn Tyr Pro Asn Met Phe Met Val Phe Gly Pro Asn Gly Pro Leu
            420                 425                 430
Ala Asn Thr Pro Pro Ile Ile Glu Ser Gln Val Glu Trp Ile Ser Val
        435                 440                 445
Phe Ile Gln Tyr Thr Val Glu Asn Asn Val Glu Ser Ile Glu Ala Asp
    450                 455                 460
Lys Glu Ala Glu Glu Gln Trp Thr Gln Thr Cys Ala Asn Ile Ala Glu
465                 470                 475                 480
Lys Thr Leu Phe Pro Lys Ala Lys Cys Arg Ile Phe Gly Ala Asn Ile
                485                 490                 495
Pro Gly Lys Lys Asn Thr Val Tyr Leu Tyr Leu Gly Gly Leu Lys Glu
            500                 505                 510
Tyr Arg Ser Val Leu Ala Asn Cys Lys Asn His Ala Tyr Val Gly Phe
        515                 520                 525
Asp Ile Gln Leu Gln Arg Ser Asp Thr Lys Gln Gln Ala Asn Ala
    530                 535                 540

<210>  5
<211>  1632
<212>  DNA
<213>  Artificial synthesis

<400>  5
atgagtacca agatggattt tgatgcaatt gtgatcggtg ccggctttgg cggcctgtat      60
gccgttaaaa aactgcgcga tgaactggaa ctgaaagtta aagcctttga taaagcaacg     120
gatgtgggcg ggacctggta ttggaatcgc tatccgggcg cactgacgga tacggaaacc     180
catctgtatt gctattcttg ggataaagaa atgctacaga gtttagaaat caaaaagaaa     240
tatgtgcagg gcccagatgt tcgcaaatac ttacagcagg ttgcagaaaa acatgatctg     300
aaaaaatctt atcagtttaa taccgccgtg acgagtgctc attataacga ggcggatgcc     360
ctgtgggaag tgacaaccga atatggcgat aaatataccg cacgctttct gattaccgcc     420
gtgggtctgc tgtctgcacc taattggcct aatatcaaag gcatcaatca gtttaaaggt     480

gaactgcatc atacgtcacg ctggccggat gatgtgagca tcgaaggcaa gagagtgggt 540
gtgatcggta cgggtagtac gggcgttcag gttattacag cagttgctcc attagccaaa 600
catctgaccg tgtttcagcg tagtccacag tatagtgttc cgatcggcaa tgatccactg 660
agcgaagaag atgttaagaa gattaaagat aattatgata aaatctggga tggtgtgaaa 720
aatagcgcct tagcctatgg tgtgaatgag tctacagttc cagccatgag cgtgagtgca 780
gaagaacgta aagccgtgtt tgaaaaggca tggcagacgg gtggcgggat gcgctttatg 840
tttgaaacct ttggggacat aattaccaat atggaagcta atatcgaagc acagaatttt 900
atcaaaggca aaattgcccg catcgttaaa gatcctgcca ttgcacagaa actgatgcct 960
caggatctgt atgcttgtcg cccgctgtgc gattcaggct attataatac ctttaatcgc 1020
gaaaatgttc gtctggaaga tgttaaagct aatccgatcg tggaaatcac cgaaaatggc 1080
gttaaactgg aaaatggcga ttttgtggaa ttagatatgc tgatttgcgc gaccggcttt 1140
gatgcgggcg atggcaacta caagcgcatc gacatacagg ggaaaaatgg cttagccatc 1200
aaagattatt ggaaagaagg tcctagtagc tatatgggcg ttgcggttaa caactatccg 1260
aatatgttta tggtttttgg tccgaatggt ccactggcca atactcctcc tatcatcgaa 1320
tctcaggttg agtggatttc agtgtttatc cagtataccg ttgaaaacaa tgttgaatct 1380
atcgaagccg ataaagaagc ggaagaacag tggacccaga cctgcgccaa tattgccgaa 1440
aaaaccctgt ttcctaaagc caaatgtcgc atctttggtg ccaatattcc gggcaagaaa 1500
aacacggtgt atctgtacct cggcggtctg aaagaatatc gtagcgtttt agctaattgc 1560
aaaaatcatg cgtatgtggg ctttgatatt cagttacagc gctcagatac caaacagcag 1620
gctaatgcgt aa 1632

<210> 6
<211> 543
<212> PRT
<213> Artificial synthesis

<400> 6
Met Ser Thr Lys Met Asp Phe Asp Ala Ile Val Ile Gly Ala Gly Phe
1               5                   10                  15
Gly Gly Leu Tyr Ala Val Lys Lys Leu Arg Asp Glu Leu Glu Leu Lys
            20                  25                  30
Val Lys Ala Phe Asp Lys Ala Thr Asp Val Gly Gly Thr Trp Tyr Trp
        35                  40                  45
Asn Arg Tyr Pro Gly Ala Leu Thr Asp Thr Glu Thr His Leu Tyr Cys
    50                  55                  60
Tyr Ser Trp Asp Lys Glu Met Leu Gln Ser Leu Glu Ile Lys Lys Lys
65                  70                  75                  80
Tyr Val Gln Gly Pro Asp Val Arg Lys Tyr Leu Gln Gln Val Ala Glu
                85                  90                  95
Lys His Asp Leu Lys Lys Ser Tyr Gln Phe Asn Thr Ala Val Thr Ser
            100                 105                 110
Ala His Tyr Asn Glu Ala Asp Ala Leu Trp Glu Val Thr Thr Glu Tyr
        115                 120                 125
Gly Asp Lys Tyr Thr Ala Arg Phe Leu Ile Thr Ala Val Gly Leu Leu
    130                 135                 140
Ser Ala Pro Asn Trp Pro Asn Ile Lys Gly Ile Asn Gln Phe Lys Gly
145                 150                 155                 160
Glu Leu His His Thr Ser Arg Trp Pro Asp Asp Val Ser Ile Glu Gly
                165                 170                 175
Lys Arg Val Gly Val Ile Gly Thr Gly Ser Thr Gly Val Gln Val Ile
            180                 185                 190
Thr Ala Val Ala Pro Leu Ala Lys His Leu Thr Val Phe Gln Arg Ser
        195                 200                 205
Pro Gln Tyr Ser Val Pro Ile Gly Asn Asp Pro Leu Ser Glu Glu Asp
    210                 215                 220
Val Lys Lys Ile Lys Asp Asn Tyr Asp Lys Ile Trp Asp Gly Val Lys
225                 230                 235                 240
Asn Ser Ala Leu Ala Tyr Gly Val Asn Glu Ser Thr Val Pro Ala Met
                245                 250                 255
Ser Val Ser Ala Glu Glu Arg Lys Ala Val Phe Glu Lys Ala Trp Gln
            260                 265                 270
Thr Gly Gly Gly Met Arg Phe Met Phe Glu Thr Phe Gly Asp Ile Ile
        275                 280                 285

Thr Asn Met Glu Ala Asn Ile Glu Ala Gln Asn Phe Ile Lys Gly Lys
    290                 295                 300
Ile Ala Arg Ile Val Lys Asp Pro Ala Ile Ala Gln Lys Leu Met Pro
305                 310                 315                 320
Gln Asp Leu Tyr Ala Cys Arg Pro Leu Cys Asp Ser Gly Tyr Tyr Asn
                325                 330                 335
Thr Phe Asn Arg Glu Asn Val Arg Leu Glu Asp Val Lys Ala Asn Pro
            340                 345                 350
Ile Val Glu Ile Thr Glu Asn Gly Val Lys Leu Glu Asn Gly Asp Phe
        355                 360                 365
Val Glu Leu Asp Met Leu Ile Cys Ala Thr Gly Phe Asp Ala Gly Asp
    370                 375                 380
Gly Asn Tyr Lys Arg Ile Asp Ile Gln Gly Lys Asn Gly Leu Ala Ile
385                 390                 395                 400
Lys Asp Tyr Trp Lys Glu Gly Pro Ser Ser Tyr Met Gly Val Ala Val
                405                 410                 415
Asn Asn Tyr Pro Asn Met Phe Met Val Phe Gly Pro Asn Gly Pro Leu
            420                 425                 430
Ala Asn Thr Pro Pro Ile Ile Glu Ser Gln Val Glu Trp Ile Ser Val
        435                 440                 445
Phe Ile Gln Tyr Thr Val Glu Asn Asn Val Glu Ser Ile Glu Ala Asp
    450                 455                 460
Lys Glu Ala Glu Glu Gln Trp Thr Gln Thr Cys Ala Asn Ile Ala Glu
465                 470                 475                 480
Lys Thr Leu Phe Pro Lys Ala Lys Cys Arg Ile Phe Gly Ala Asn Ile
                485                 490                 495
Pro Gly Lys Lys Asn Thr Val Tyr Leu Tyr Leu Gly Gly Leu Lys Glu
            500                 505                 510
Tyr Arg Ser Val Leu Ala Asn Cys Lys Asn His Ala Tyr Val Gly Phe
        515                 520                 525
Asp Ile Gln Leu Gln Arg Ser Asp Thr Lys Gln Gln Ala Asn Ala
    530                 535                 540

<210>  7
<211>  1626
<212>  DNA
<213>  Artificial synthesis

<400>  7
atgaccaacg cgaagaacac cgatctcgac gcgatcgtcg tcggtgccgg gttcgccggc      60
atctacgcgc tgcacaagct ccgcaacgaa ctgggcctgt ccgtccgggc cttcgacaag     120
gcgggcgggg tcgggggcac ctggtactgg aaccgctacc ccggcgccat gtccgacagc     180
gagggcttca tctaccagta ctccttcgac cgcgacctgc tgcgggagtg gacctggaag     240
aagcgctacc tgtcccaggc ggagatcctg ggctacctgg aggcggtcgt ggagcggcac     300
gacctcgcca gggacatcca gctcaacacc ggcgtcgaga cgctggtcta cgacgaggcc     360
gccgccctgt ggaccgcgac caccagcgac ggccagaccc tcaccgcccg ctacgtggtg     420
accgccctcg gaccgctgtc cacctcccac ttccccgact tcaagggccg cgacagcttc     480
cagggccgcc tggtccacac cggctcctgg cccgacgacc tcgacatcga gggcaagagg     540
gtcggcgtca tcggcaccgg ctccaccgga acccagttca tctgcgcggc ctcgaaggtg     600
gccgggcagc tcaccgtgtt ccagcggacc ccccagtaca acgtgccctc gggcaacgcc     660
gaggtggacg aggcctactt caccgacctg cgcggccgct acgaccaggt ctgggagcag     720
gccaagaagt cccgcgtggc gtgcggcttc gaggagagcg agatcgccgc gatgagcgtc     780
tccgaggagg agcgccgacg cgtcttccag gagaactggg accggggcaa cggcttccgc     840
ttcatgttcg gcaccttctc cgacatcatc ttcgacccca gggccaacga ggcggcggcc     900
gacttcatcc ggtccaagat ccgggagatc gtcaaggacc cagagaccgc ccgcaagctc     960
cagccgaccg actactacgc caagcgcccg gtctgcaacg aggactacta cgagtcctac    1020
aaccgggaca acgtcagcct ggtgagcctc aaggagaccc cgatccggga gttcaccccc    1080
acggggatcg tgaccgagga cggggtggag cacgagctgg acgtcgtcgt cttcgcgacc    1140
ggtttcgagg ccgtcgaggg cagctaccgg cagatggaga tccgcggccg gggcggcgtg    1200
accatcgagg agcactgggg ggacacgccc gccagctacc tcggggtcaa cgtctcgggc    1260
ttccccaaca tgttcatggt ctacggcccc aacagcgtct tcagcaacct gcccacggcc    1320
atcgagaccc aggtcgagtg gatcaccgac ctggtccgga tgatggagga gcgcgacctg    1380
acctccatcg agccgacccc ggaggcggag gagggctgga ccgagctgtg cacgcagatc    1440

gccgaccact ccctgttccc caaggtcaac tcctggatct tcggggccaa catcccgggc 1500
aagaagaagc gggtcctgtt ctacttcgcg gggctcggca actaccgcca gaagctcggt 1560
gacgtggccg cggccgacta cgagggcttc atgctcaagg gcaacccctc ggtggtgacc 1620
gcctga 1626

<210> 8
<211> 541
<212> PRT
<213> Artificial synthesis

<400> 8

Met Thr Asn Ala Lys Asn Thr Asp Leu Asp Ala Ile Val Val Gly Ala
1               5                   10                  15

Gly Phe Ala Gly Ile Tyr Ala Leu His Lys Leu Arg Asn Glu Leu Gly
            20                  25                  30

Leu Ser Val Arg Ala Phe Asp Lys Ala Gly Gly Val Gly Gly Thr Trp
        35                  40                  45

Tyr Trp Asn Arg Tyr Pro Gly Ala Met Ser Asp Ser Glu Gly Phe Ile
    50                  55                  60

Tyr Gln Tyr Ser Phe Asp Arg Asp Leu Leu Arg Glu Trp Thr Trp Lys
65                  70                  75                  80

Lys Arg Tyr Leu Ser Gln Ala Glu Ile Leu Gly Tyr Leu Glu Ala Val
                85                  90                  95

Val Glu Arg His Asp Leu Ala Arg Asp Ile Gln Leu Asn Thr Gly Val
            100                 105                 110

Glu Thr Leu Val Tyr Asp Glu Ala Ala Ala Leu Trp Thr Ala Thr Thr
        115                 120                 125

Ser Asp Gly Gln Thr Leu Thr Ala Arg Tyr Val Val Thr Ala Leu Gly
    130                 135                 140

Pro Leu Ser Thr Ser His Phe Pro Asp Phe Lys Gly Arg Asp Ser Phe
145                 150                 155                 160

Gln Gly Arg Leu Val His Thr Gly Ser Trp Pro Asp Asp Leu Asp Ile
                165                 170                 175

Glu Gly Lys Arg Val Gly Val Ile Gly Thr Gly Ser Thr Gly Thr Gln
            180                 185                 190

Phe Ile Cys Ala Ala Ser Lys Val Ala Gly Gln Leu Thr Val Phe Gln
        195                 200                 205

Arg Thr Pro Gln Tyr Asn Val Pro Ser Gly Asn Ala Glu Val Asp Glu
    210                 215                 220

Ala Tyr Phe Thr Asp Leu Arg Gly Arg Tyr Asp Gln Val Trp Glu Gln
225                 230                 235                 240

Ala Lys Lys Ser Arg Val Ala Cys Gly Phe Glu Glu Ser Glu Ile Ala
                245                 250                 255

Ala Met Ser Val Ser Glu Glu Glu Arg Arg Arg Val Phe Gln Glu Asn
            260                 265                 270

Trp Asp Arg Gly Asn Gly Phe Arg Phe Met Phe Gly Thr Phe Ser Asp
        275                 280                 285

Ile Ile Phe Asp Pro Arg Ala Asn Glu Ala Ala Ala Asp Phe Ile Arg
    290                 295                 300

Ser Lys Ile Arg Glu Ile Val Lys Asp Pro Glu Thr Ala Arg Lys Leu
305                 310                 315                 320

Gln Pro Thr Asp Tyr Tyr Ala Lys Arg Pro Val Cys Asn Glu Asp Tyr
                325                 330                 335

Tyr Glu Ser Tyr Asn Arg Asp Asn Val Ser Leu Val Ser Leu Lys Glu
            340                 345                 350

Thr Pro Ile Arg Glu Phe Thr Pro Thr Gly Ile Val Thr Glu Asp Gly
        355                 360                 365

Val Glu His Glu Leu Asp Val Val Val Phe Ala Thr Gly Phe Glu Ala
    370                 375                 380

Val Glu Gly Ser Tyr Arg Gln Met Glu Ile Arg Gly Arg Gly Gly Val
385                 390                 395                 400

Thr Ile Glu Glu His Trp Gly Asp Thr Pro Ala Ser Tyr Leu Gly Val
                405                 410                 415

Asn Val Ser Gly Phe Pro Asn Met Phe Met Val Tyr Gly Pro Asn Ser
            420                 425                 430

Val Phe Ser Asn Leu Pro Thr Ala Ile Glu Thr Gln Val Glu Trp Ile
        435                 440                 445

Thr Asp Leu Val Arg Met Met Glu Glu Arg Asp Leu Thr Ser Ile Glu
    450                 455                 460

Pro Thr Pro Glu Ala Glu Glu Gly Trp Thr Glu Leu Cys Thr Gln Ile
465                 470                 475                 480

Ala Asp His Ser Leu Phe Pro Lys Val Asn Ser Trp Ile Phe Gly Ala
                485                 490                 495

Asn Ile Pro Gly Lys Lys Lys Arg Val Leu Phe Tyr Phe Ala Gly Leu
            500                 505                 510

Gly Asn Tyr Arg Gln Lys Leu Gly Asp Val Ala Ala Ala Asp Tyr Glu
        515                 520                 525

Gly Phe Met Leu Lys Gly Asn Pro Ser Val Val Thr Ala
    530                 535                 540

<210>  9
<211>  1635
<212>  DNA
<213>  Artificial synthesis

<400>  9
atgagcacga ccaaagcgaa gcgagcggat aatggcgggg cgaaagaact cgacgttctt      60

gttgtgggcg cgggatttgc tggtgtttac cttcttgacc gcctgcgcgg tatgggcatg     120

tcggtccaag cgattgaggc tgggagcggc cttggaggtg tctggtactg gaattgttat     180

ccaggagcgc gcgtcgattc tcccggcccg atgtatcagt tttcgcgcga ggatctgtgg 240

cgagattgga agttcacgga gctctatcct gcgtggcctg aaatccgtga ctacttccac 300

tatctggacg agaagctcga ccttagccgc gacattcgct tcaacgcgcg cgtcgaggag 360

gctgagtttg acgcggcgag caatcgttgg acggtccgtt cgagcgacgg ctccgtaatc 420

cggccccgtt acttcgtggt ctgcgttggt ttgggttcga agccctacac accgaatctg 480

ccgggcctga gcgactttgc cggggaacga catcacaccg cgctttggcc gcagcaaggt 540

ttggacttgg ccggcaagcg ggtcggggtc atcggcacag ggcaagcggc cgtgcaggtg 600

gctcaagaag ccgccgcagt ggcggcccaa ctcacggtct tccagcgcac gcctaatctg 660

gctctgccga tgcggcagaa gaggctggac gatgacacaa ttcttcgcat gaagaaggga 720

tacccggtcg cctacgagaa gcgcagaacg acgcttggag gtttcgacta ccagtctctc 780

gagaaggccg cgtcggaggt atccgccgag gagcggcagg cgacgtttga gcgcgtttgg 840

gagatcggcg gctttgcgcc gtgggttggc tcgttcaatg accttcttat cgacgagcag 900

tcgaaccgcg ccgcctacga tttttggcgt gacaagacgc gggctcggat caaggatcca 960

gccattgccg agatcctggc gccgacggag ccgattcatc cctatggcgc caagcggccg 1020

tcgttggagc agaatttctt cgacatcttc aatcagtcca acgtgagcct tgtcgatctc 1080

cgcaacacgc cgatcgagaa agtgacccgc agtgggatca agactacagc cggcgagtac 1140

ggactagatg ttctggtcct ggctaccggg tttgacgcgg tgacaggtgg actgaccagc 1200

atcgatatct ggggcacgga cggcaggact ctgaagcaaa agtgggcgga cggcgttcga 1260

gcccatctgg gaatggcatc cgccggttat cccaatctcc tttttgtcta tggtccgcac 1320

agcccgaacg ctttcgccaa tggaccgact gccgcggagc ttcagggcga gtgggtcgcc 1380

aagatgctgg accatgtacg cggacgcaac tgggcgcagt tcgaggcgac ggtgccggcg 1440

gaggaagcgt ggcgtgcgaa agtgtttgaa gcggtggacg ccactctcat cccccgcgcc 1500

gactcctggt gggtcggcgc gaacataccc ggcaagcggc gagagatgct ggcctttgct 1560

ggcggtctcg ggacgtacat ggcgatatgc aacgaaagcg ccgagcgcgg gtatgaggga 1620

ttttcgatcg gctga 1635

<210> 10
<211> 544
<212> PRT
<213> Artificial synthesis

<400> 10

Met Ser Thr Thr Lys Ala Lys Arg Ala Asp Asn Gly Gly Ala Lys Glu
1               5                   10                  15

Leu Asp Val Leu Val Val Gly Ala Gly Phe Ala Gly Val Tyr Leu Leu
            20                  25                  30

Asp Arg Leu Arg Gly Met Gly Met Ser Val Gln Ala Ile Glu Ala Gly
        35                  40                  45

Ser Gly Leu Gly Gly Val Trp Tyr Trp Asn Cys Tyr Pro Gly Ala Arg
    50                  55                  60

Val Asp Ser Pro Gly Pro Met Tyr Gln Phe Ser Arg Glu Asp Leu Trp
65                  70                  75                  80

Arg Asp Trp Lys Phe Thr Glu Leu Tyr Pro Ala Trp Pro Glu Ile Arg
                85                  90                  95

Asp Tyr Phe His Tyr Leu Asp Glu Lys Leu Asp Leu Ser Arg Asp Ile
            100                 105                 110

Arg Phe Asn Ala Arg Val Glu Glu Ala Glu Phe Asp Ala Ala Ser Asn
        115                 120                 125

Arg Trp Thr Val Arg Ser Ser Asp Gly Ser Val Ile Arg Pro Arg Tyr
    130                 135                 140

Phe Val Val Cys Val Gly Leu Gly Ser Lys Pro Tyr Thr Pro Asn Leu
145                 150                 155                 160

Pro Gly Leu Ser Asp Phe Ala Gly Glu Arg His His Thr Ala Leu Trp
                165                 170                 175

Pro Gln Gln Gly Leu Asp Leu Ala Gly Lys Arg Val Gly Val Ile Gly
            180                 185                 190

Thr Gly Ala Ser Gly Val Gln Val Ala Gln Glu Ala Ala Ala Val Ala
        195                 200                 205

Ala Gln Leu Thr Val Phe Gln Arg Thr Pro Asn Leu Ala Leu Pro Met
    210                 215                 220

Arg Gln Lys Arg Leu Asp Asp Asp Thr Ile Leu Arg Met Lys Lys Gly
225                 230                 235                 240

Tyr Pro Val Ala Tyr Glu Lys Arg Arg Thr Thr Leu Gly Gly Phe Asp
                245                 250                 255

Tyr Gln Ser Leu Glu Lys Ala Ala Ser Glu Val Ser Ala Glu Glu Arg
            260                 265                 270

Gln Ala Thr Phe Glu Arg Val Trp Glu Ile Gly Gly Phe Ala Pro Trp
        275                 280                 285

Val Gly Ser Phe Asn Asp Leu Leu Ile Asp Glu Gln Ser Asn Arg Ala
    290                 295                 300

Ala Tyr Asp Phe Trp Arg Asp Lys Thr Arg Ala Arg Ile Lys Asp Pro
305                 310                 315                 320

Ala Ile Ala Glu Ile Leu Ala Pro Thr Glu Pro Ile His Pro Tyr Gly
                325                 330                 335

Ala Lys Arg Pro Ser Leu Glu Gln Asn Phe Phe Asp Ile Phe Asn Gln
            340                 345                 350

Ser Asn Val Ser Leu Val Asp Leu Arg Asn Thr Pro Ile Glu Lys Val
        355                 360                 365

Thr Arg Ser Gly Ile Lys Thr Thr Ala Gly Glu Tyr Gly Leu Asp Val
    370                 375                 380

Leu Val Leu Ala Thr Gly Phe Asp Ala Val Thr Gly Gly Leu Thr Ser
385                 390                 395                 400

Ile Asp Ile Trp Gly Thr Asp Gly Arg Thr Leu Lys Gln Lys Trp Ala
                405                 410                 415

Asp Gly Val Arg Ala His Leu Gly Met Ala Ser Ala Gly Tyr Pro Asn
            420                 425                 430

Leu Leu Phe Val Tyr Gly Pro His Ser Pro Asn Ala Phe Ala Asn Gly
        435                 440                 445

Pro Thr Ala Ala Glu Leu Gln Gly Glu Trp Val Ala Lys Met Leu Asp
    450                 455                 460

His Val Arg Gly Arg Asn Trp Ala Gln Phe Glu Ala Thr Val Pro Ala
465                 470                 475                 480

Glu Glu Ala Trp Arg Ala Lys Val Phe Glu Ala Val Asp Ala Thr Leu
                485                 490                 495

Ile Pro Arg Ala Asp Ser Trp Trp Val Gly Ala Asn Ile Pro Gly Lys
            500                 505                 510

Arg Arg Glu Met Leu Ala Phe Ala Gly Gly Leu Gly Thr Tyr Met Ala
        515                 520                 525

Ile Cys Asn Glu Ser Ala Glu Arg Gly Tyr Glu Gly Phe Ser Ile Gly
    530                 535                 540

<210>  11
<211>  1632
<212>  DNA
<213>  Artificial synthesis

<400>  11
atgaaccccg agaccccatc ggcaccagcg caggaccagg ccgacgtcga cgtggtcgtc      60

gtcggcgcgg gcatcgccgg gctgtacgcc ctgcaccgat tccgcggcag cggcctgacg     120

gtgcgggtgt tcgaagcagg ccagggcgtg ggcggcgtct ggtactggaa ccgctacccg     180

ggcgcgcgct gcgacgtgga gagcgtcgac tactcgtact ccttcgacga ggccctgcag     240

caggagtggg actggagcga gaagtacgcc acccagcccg agatcctgtc ctacctcgag     300

cacgtcgccg accgctacga cctgcgccgc gacatcgaat tcggtacccg cgtcaccgat     360

gtcgtgctcg acgagacgac cctgcgctgg caggtcgcca ccgacaccgg ccgcacggtg     420

cgggcccggt tcgtggtgct ggccaccggg ccgctgtcca acgccaacac cccggccatc     480

gaggggctgg acaccttcgc cgggcaggtg ctgcacacct cgcactggcc gcacgaaggc     540

gtcgacctga ccggcaggcg ggtcggcgtg atcggcaccg gatcctccgg catccagacg     600

atcccgtgcg tggccgagca ggccgcgcag ctgcacgtgt tccagcgcac cgccaactac     660

agtgtgcccg cgggcaatac gccgctcacc gacgacgacc ggcgggcaca gaaggcgaac     720

tatcccgagc gcaggcggct gtcgatggcc agcggcggcg gctcgccgca ccaggcccat 780

cccgagtccg cgctcgcggt ggacgcgggg gaactccgcg ccgcctacga gaagcgctgg 840

gcgctgggcg gtgtgctgtt cagcaagacc ttcccggacc agctggtcac cctggccgcc 900

aacgacaccg cgcgcgagtt ctgggaggag aaggtgcgcg cggtcatcga cgacccgcgg 960

gtggccgagc tgctggtgcc gcgcgatcac ccgatcggca ccaagcgcat ctgcaccgac 1020

acgaactact accagaccta caaccgcgac aatgtcgagc tggtcgacct gcgggccacg 1080

cccatcaccc gggtcgacgc cgcgggcatc cacaccaccg acgcgcacta cccgctcgac 1140

accctggtcc tggccaccgg cttcgacgcg atgaccggct cggtgtcgcg gatgaacatc 1200

gtgggccgcg gcggtgagac gctcaacgag gcgtggcgcg aagggccgaa aacctatctg 1260

ggactgggca tgacgggctt cccgaacctg ttcaacctca ccggacccgg cagtccgtcg 1320

gtgctggcca acatggtgct gcactcggaa ctgcacgtgg actgggtggc cgaggccatc 1380

gccttcctcg acggccgggg cgcgctggcg ctcgaggcac ggccggacgc ggtcgccgac 1440

tgggtccgcg agtgcgcgcg gcgggccgag ggaaccctga tgatgcaggc gaattcgtgg 1500

tacctgggcg ccaatatcga gggcaggcca cgggtcttca tgcccttcgt cggcgggttc 1560

ggggtctacg gcgagatcat cgccgaggtc gcggccgcgg gctacaaggg cttcgacgtc 1620

ctggaggact ga 1632

<210> 12
<211> 543
<212> PRT
<213> Artificial synthesis

<400> 12

Met Asn Pro Glu Thr Pro Ser Ala Pro Ala Gln Asp Gln Ala Asp Val
1               5                   10                  15

Asp Val Val Val Val Gly Ala Gly Ile Ala Gly Leu Tyr Ala Leu His
            20                  25                  30

Arg Phe Arg Gly Ser Gly Leu Thr Val Arg Val Phe Glu Ala Gly Gln
        35                  40                  45

Gly Val Gly Gly Val Trp Tyr Trp Asn Arg Tyr Pro Gly Ala Arg Cys
    50                  55                  60

Asp Val Glu Ser Val Asp Tyr Ser Tyr Ser Phe Asp Glu Ala Leu Gln
65                  70                  75                  80

Gln Glu Trp Asp Trp Ser Glu Lys Tyr Ala Thr Gln Pro Glu Ile Leu
                85                  90                  95

Ser Tyr Leu Glu His Val Ala Asp Arg Tyr Asp Leu Arg Arg Asp Ile
            100                 105                 110

Glu Phe Gly Thr Arg Val Thr Asp Val Val Leu Asp Glu Thr Thr Leu
        115                 120                 125

Arg Trp Gln Val Ala Thr Asp Thr Gly Arg Thr Val Arg Ala Arg Phe
    130                 135                 140

Val Val Leu Ala Thr Gly Pro Leu Ser Asn Ala Asn Thr Pro Ala Ile
145                 150                 155                 160

Glu Gly Leu Asp Thr Phe Ala Gly Gln Val Leu His Thr Ser His Trp
                165                 170                 175

Pro His Glu Gly Val Asp Leu Thr Gly Arg Arg Val Gly Val Ile Gly
            180                 185                 190

Thr Gly Ser Ser Gly Ile Gln Thr Ile Pro Cys Val Ala Glu Gln Ala
        195                 200                 205

Ala Gln Leu His Val Phe Gln Arg Thr Ala Asn Tyr Ser Val Pro Ala
    210                 215                 220

Gly Asn Thr Pro Leu Thr Asp Asp Asp Arg Arg Ala Gln Lys Ala Asn
225                 230                 235                 240

Tyr Pro Glu Arg Arg Arg Leu Ser Met Ala Ser Gly Gly Gly Ser Pro
                245                 250                 255

His Gln Ala His Pro Glu Ser Ala Leu Ala Val Asp Ala Gly Glu Leu
            260                 265                 270

Arg Ala Ala Tyr Glu Lys Arg Trp Ala Leu Gly Gly Val Leu Phe Ser
        275                 280                 285

Lys Thr Phe Pro Asp Gln Leu Val Thr Leu Ala Ala Asn Asp Thr Ala
    290                 295                 300

Arg Glu Phe Trp Glu Glu Lys Val Arg Ala Val Ile Asp Asp Pro Arg
305                 310                 315                 320

Val Ala Glu Leu Leu Val Pro Arg Asp His Pro Ile Gly Thr Lys Arg
                325                 330                 335

Ile Cys Thr Asp Thr Asn Tyr Tyr Gln Thr Tyr Asn Arg Asp Asn Val
            340                 345                 350

Glu Leu Val Asp Leu Arg Ala Thr Pro Ile Thr Arg Val Asp Ala Ala
        355                 360                 365

Gly Ile His Thr Thr Asp Ala His Tyr Pro Leu Asp Thr Leu Val Leu
    370                 375                 380

Ala Thr Gly Phe Asp Ala Met Thr Gly Ser Val Ser Arg Met Asn Ile
385                 390                 395                 400

Val Gly Arg Gly Gly Glu Thr Leu Asn Glu Ala Trp Arg Glu Gly Pro
                405                 410                 415

Lys Thr Tyr Leu Gly Leu Gly Met Thr Gly Phe Pro Asn Leu Phe Asn
            420                 425                 430

Leu Thr Gly Pro Gly Ser Pro Ser Val Leu Ala Asn Met Val Leu His
        435                 440                 445

Ser Glu Leu His Val Asp Trp Val Ala Glu Ala Ile Ala Phe Leu Asp
    450                 455                 460

Gly Arg Gly Ala Leu Ala Leu Glu Ala Arg Pro Asp Ala Val Ala Asp
465                 470                 475                 480

Trp Val Arg Glu Cys Ala Arg Arg Ala Glu Gly Thr Leu Met Met Gln
                485                 490                 495

Ala Asn Ser Trp Tyr Leu Gly Ala Asn Ile Glu Gly Arg Pro Arg Val
            500                 505                 510

Phe Met Pro Phe Val Gly Gly Phe Gly Val Tyr Gly Glu Ile Ile Ala
        515                 520                 525

Glu Val Ala Ala Ala Gly Tyr Lys Gly Phe Asp Val Leu Glu Asp
    530                 535                 540

<210>  13
<211>  759
<212>  DNA
<213>  Artificial synthesis

<400>  13
atgacggatc gtctgaaagg caaagttgcc atcgtgaccg gtggcacgct gggcatcggc      60
ctggccattg ccgataaatt tgtggaagaa ggcgccaaag tggttattac aggtcgtcat     120
gcagatgtgg gcgaaaaagc cgccaaatct atcggcggca ccgatgttat tcgctttgtg     180
cagcatgatg cgtcagatga agcgggttgg acgaaactgt ttgatacaac ggaagaagcc     240
tttggtccag tgacaacggt tgtgaataac gccggtatct ttgtgtacaa gtcagtggaa     300
gatacgacga cagaagaatg gcgcaaactg ctgagtgtta acctggatgg cgtgtttttc     360
ggtacacgtt taggtatcca gcgcatgaaa aataagggct taggtgcgag tatcatcaat     420
atgtcctcaa tcgaaggctt agtgggcgat cctacgggtg gtgcttataa tgcctctaaa     480
ggtgcagttc gcattatgtc taaatcagct gcactggatt gcgctctgaa agattatgat     540
gttcgcgtta ataccgttca tccgggctat atcaaaacac cattagttga tgatctggaa     600
ggtgccgaag aaatgatgtc tcagcgtacc aaaaccccta tgggtcatat cggtgaacct     660
aatgatattg cttggatctg cgtgtatctg gcgtcagatg aatctaaatt tgcaacaggc     720
gcggaatttg tggttgatgg cggctatacc gcccagtaa                            759

<210>  14
<211>  252
<212>  PRT
<213>  Artificial synthesis

<400>  14
Met Thr Asp Arg Leu Lys Gly Lys Val Ala Ile Val Thr Gly Gly Thr
1               5                   10                  15
Leu Gly Ile Gly Leu Ala Ile Ala Asp Lys Phe Val Glu Glu Gly Ala
            20                  25                  30
Lys Val Val Ile Thr Gly Arg His Ala Asp Val Gly Glu Lys Ala Ala
        35                  40                  45
Lys Ser Ile Gly Gly Thr Asp Val Ile Arg Phe Val Gln His Asp Ala
    50                  55                  60
Ser Asp Glu Ala Gly Trp Thr Lys Leu Phe Asp Thr Thr Glu Glu Ala
65                  70                  75                  80
Phe Gly Pro Val Thr Thr Val Val Asn Asn Ala Gly Ile Phe Val Tyr
                85                  90                  95
Lys Ser Val Glu Asp Thr Thr Thr Glu Glu Trp Arg Lys Leu Leu Ser
            100                 105                 110
Val Asn Leu Asp Gly Val Phe Phe Gly Thr Arg Leu Gly Ile Gln Arg
        115                 120                 125
Met Lys Asn Lys Gly Leu Gly Ala Ser Ile Ile Asn Met Ser Ser Ile
    130                 135                 140
Glu Gly Leu Val Gly Asp Pro Thr Gly Gly Ala Tyr Asn Ala Ser Lys
145                 150                 155                 160
Gly Ala Val Arg Ile Met Ser Lys Ser Ala Ala Leu Asp Cys Ala Leu
                165                 170                 175

Lys Asp Tyr Asp Val Arg Val Asn Thr Val His Pro Gly Tyr Ile Lys
            180                 185                 190
Thr Pro Leu Val Asp Asp Leu Glu Gly Ala Glu Glu Met Met Ser Gln
        195                 200                 205
Arg Thr Lys Thr Pro Met Gly His Ile Gly Glu Pro Asn Asp Ile Ala
    210                 215                 220
Trp Ile Cys Val Tyr Leu Ala Ser Asp Glu Ser Lys Phe Ala Thr Gly
225                 230                 235                 240
Ala Glu Phe Val Val Asp Gly Gly Tyr Thr Ala Gln
                245                 250

<210>  15
<211>  759
<212>  DNA
<213>  Artificial synthesis

<400>  15
atgactgatc gtttaaaagg caaagtagca attgtaactg gcggtacctt gggaattggc      60
ttggcaatcg ctgataagtt tgttgaagaa ggcgcaaagg ttgttattac cggccgtcac     120
gctgatgtag gtgaaaaagc tgccaaatca atcggcggca cagacgttat ccgttttgtc     180
caacacgatg cttctgatga agccggctgg actaagttgt ttgatacgac tgaagaagca     240
tttggcccag ttaccacggt tgtcaacaat gccggaattg cggtcagcaa gagtgttgaa     300
gataccacaa ctgaagaatg gcgcaagctg ctctcagtta acttggatgg tgtcttcttc     360
ggtacccgtc ttggaatcca acgtatgaag aataaaggac tcggagcatc aatcatcaat     420
atgtcatcta tcgaaggttt tgttggtgat ccaactctgg gtgcatacaa cgcttcaaaa     480
ggtgctgtca gaattatgtc taaatcagct gccttggatt gcgctttgaa ggactacgat     540
gttcgggtta acactgttca tccaggttat atcaagacac cattggttga cgatcttgaa     600
ggggcagaag aaatgatgtc acagcggacc aagacaccaa tgggtcatat cggtgaacct     660
aacgatatcg cttggatctg tgtttacctg gcatctgacg aatctaaatt tgccactggt     720
gcagaattcg ttgtcgatgg tggatacact gctcaataa                            759

<210>  16
<211>  252
<212>  PRT
<213>  Artificial synthesis

<400>  16
Met Thr Asp Arg Leu Lys Gly Lys Val Ala Ile Val Thr Gly Gly Thr
1               5                   10                  15
Leu Gly Ile Gly Leu Ala Ile Ala Asp Lys Phe Val Glu Glu Gly Ala
            20                  25                  30
Lys Val Val Ile Thr Gly Arg His Ala Asp Val Gly Glu Lys Ala Ala
        35                  40                  45
Lys Ser Ile Gly Gly Thr Asp Val Ile Arg Phe Val Gln His Asp Ala
    50                  55                  60
Ser Asp Glu Ala Gly Trp Thr Lys Leu Phe Asp Thr Thr Glu Glu Ala
65                  70                  75                  80
Phe Gly Pro Val Thr Thr Val Val Asn Asn Ala Gly Ile Ala Val Ser
                85                  90                  95
Lys Ser Val Glu Asp Thr Thr Thr Glu Glu Trp Arg Lys Leu Leu Ser
            100                 105                 110
Val Asn Leu Asp Gly Val Phe Phe Gly Thr Arg Leu Gly Ile Gln Arg
        115                 120                 125
Met Lys Asn Lys Gly Leu Gly Ala Ser Ile Ile Asn Met Ser Ser Ile
    130                 135                 140
Glu Gly Phe Val Gly Asp Pro Thr Leu Gly Ala Tyr Asn Ala Ser Lys
145                 150                 155                 160
Gly Ala Val Arg Ile Met Ser Lys Ser Ala Ala Leu Asp Cys Ala Leu
                165                 170                 175
Lys Asp Tyr Asp Val Arg Val Asn Thr Val His Pro Gly Tyr Ile Lys
            180                 185                 190
Thr Pro Leu Val Asp Asp Leu Glu Gly Ala Glu Glu Met Met Ser Gln
        195                 200                 205

Arg Thr Lys Thr Pro Met Gly His Ile Gly Glu Pro Asn Asp Ile Ala
    210                 215                 220
Trp Ile Cys Val Tyr Leu Ala Ser Asp Glu Ser Lys Phe Ala Thr Gly
225                 230                 235                 240
Ala Glu Phe Val Val Asp Gly Gly Tyr Thr Ala Gln
                245                 250

<210>  17
<211>  759
<212>  DNA
<213>  Artificial synthesis

<400>  17
atgacggatc gtctgaaagg caaagttgcc atcgtgaccg gtggcacgct gggcatcggc      60
ctggccattg ccgataaatt tgtggaagaa ggcgccaaag tggttattac aggtcgtcat     120
gcagatgtgg gcgaaaaagc cgccaaatct atcggcggca ccgatgttat tcgctttgtg     180
cagcatgatg cgtcagatga agcgggttgg acgaaactgt ttgatacaac ggaagaagcc     240
tttggtccag tgacaacggt tgtgaataac gccggtatct ttgtgagcaa gtcagtggaa     300
gatacgacga cagaagaatg gcgcaaactg ctgagtgtta acctggatgg cgtgtttttc     360
ggtacacgtt taggtatcca gcgcatgaaa aataagggct taggtgcgag tatcatcaat     420
atgtcctcaa tcgaaggctt cgtgggcgat cctacgctcg gtgcttataa tgcctctaaa     480
ggtgcagttc gcattatgtc taaatcagct gcactggatt gcgctctgaa agattatgat     540
gttcgcgtta ataccgttca tccgggctat atcaaaacac cattagttga tgatctggaa     600
ggtgccgaag aaatgatgtc tcagcgtacc aaaaccccta tgggtcatat cggtgaacct     660
aatgatattg cttggatctg cgtgtatctg gcgtcagatg aatctaaatt tgcaacaggc     720
gcggaatttg tggttgatgg cggctatacc gcccagtaa                            759

<210>  18
<211>  252
<212>  PRT
<213>  Artificial synthesis

<400>  18
Met Thr Asp Arg Leu Lys Gly Lys Val Ala Ile Val Thr Gly Gly Thr
1               5                   10                  15
Leu Gly Ile Gly Leu Ala Ile Ala Asp Lys Phe Val Glu Glu Gly Ala
            20                  25                  30
Lys Val Val Ile Thr Gly Arg His Ala Asp Val Gly Glu Lys Ala Ala
        35                  40                  45
Lys Ser Ile Gly Gly Thr Asp Val Ile Arg Phe Val Gln His Asp Ala
    50                  55                  60
Ser Asp Glu Ala Gly Trp Thr Lys Leu Phe Asp Thr Thr Glu Glu Ala
65                  70                  75                  80
Phe Gly Pro Val Thr Thr Val Val Asn Asn Ala Gly Ile Phe Val Ser
                85                  90                  95
Lys Ser Val Glu Asp Thr Thr Thr Glu Glu Trp Arg Lys Leu Leu Ser
            100                 105                 110
Val Asn Leu Asp Gly Val Phe Phe Gly Thr Arg Leu Gly Ile Gln Arg
        115                 120                 125
Met Lys Asn Lys Gly Leu Gly Ala Ser Ile Ile Asn Met Ser Ser Ile
    130                 135                 140
Glu Gly Phe Val Gly Asp Pro Thr Leu Gly Ala Tyr Asn Ala Ser Lys
145                 150                 155                 160
Gly Ala Val Arg Ile Met Ser Lys Ser Ala Ala Leu Asp Cys Ala Leu
                165                 170                 175
Lys Asp Tyr Asp Val Arg Val Asn Thr Val His Pro Gly Tyr Ile Lys
            180                 185                 190
Thr Pro Leu Val Asp Asp Leu Glu Gly Ala Glu Glu Met Met Ser Gln
        195                 200                 205
Arg Thr Lys Thr Pro Met Gly His Ile Gly Glu Pro Asn Asp Ile Ala
    210                 215                 220
Trp Ile Cys Val Tyr Leu Ala Ser Asp Glu Ser Lys Phe Ala Thr Gly
225                 230                 235                 240

Ala Glu Phe Val Val Asp Gly Gly Tyr Thr Ala Gln
                245                 250

<210>  19
<211>  768
<212>  DNA
<213>  Artificial synthesis

<400>  19
atgtccccga accagtccca cgagacgttc tccggtcacg ttgtgatcgt caccggcgcg      60

ggaaccggca tcggccgagc caccgccgtg gccttcgccg agtcgggtgc gcacgtcctc     120

gccgtgggcc gccgcgaggc gctcctgaag gagaccgccg cggcccactc ccatatcgac     180

gtcctggcca tcgacatctg cggtgacgag gcgcccggca aggtggtgga cgccgccgtg     240

gagcggtggg gacgcctcga ccacctgatc aacaacgccg gtgcgacagc ggtgatgccg     300

ctggccgagg ccgagaagca gacgatcgtc gatctgctgg cgctcaacgt cgtcgcgccc     360

agtcttctgg cgcgcgaagc cctgccgcac ctgcgccgga cctcggggtc gatcatcaac     420

ctctccagca cctacgggca ccgcccgatg gcggggggtg cgcactactc ggccaccaag     480

gccgccgtcg agcagatgac gcggagctgg gcactggaac tggccgacga gggtgtccgt     540

gtcaactcgg tcgctcccgg ccccacccgc accgacgtca tgctgcacgc cggtctgacg     600

ccggaggcgg ccaacgacat gtacgcgtac gagcgcgacc gcatcccgac ccaccacatc     660

gcgcacgcgg acgaggtggc tcactggatc ctccgtatgg cggacccggc cggacgccat     720

gccaccggtc aggtgatcac cgtcgacggc ggcctggaac tcatctga                  768

<210>  20
<211>  255
<212>  PRT
<213>  Artificial synthesis

<400>  20

Met Ser Pro Asn Gln Ser His Glu Thr Phe Ser Gly His Val Val Ile
1               5                   10                  15

Val Thr Gly Ala Gly Thr Gly Ile Gly Arg Ala Thr Ala Val Ala Phe
            20                  25                  30

Ala Glu Ser Gly Ala His Val Leu Ala Val Gly Arg Arg Glu Ala Leu
        35                  40                  45

Leu Lys Glu Thr Ala Ala Ala His Ser His Ile Asp Val Leu Ala Ile
    50                  55                  60

Asp Ile Cys Gly Asp Glu Ala Pro Gly Lys Val Val Asp Ala Ala Val
65                  70                  75                  80

Glu Arg Trp Gly Arg Leu Asp His Leu Ile Asn Asn Ala Gly Ala Thr
                85                  90                  95

Ala Val Met Pro Leu Ala Glu Ala Glu Lys Gln Thr Ile Val Asp Leu
            100                 105                 110

Leu Ala Leu Asn Val Val Ala Pro Ser Leu Leu Ala Arg Glu Ala Leu
        115                 120                 125

Pro His Leu Arg Arg Thr Ser Gly Ser Ile Ile Asn Leu Ser Ser Thr
    130                 135                 140

Tyr Gly His Arg Pro Met Ala Gly Gly Ala His Tyr Ser Ala Thr Lys
145                 150                 155                 160

Ala Ala Val Glu Gln Met Thr Arg Ser Trp Ala Leu Glu Leu Ala Asp
                165                 170                 175

Glu Gly Val Arg Val Asn Ser Val Ala Pro Gly Pro Thr Arg Thr Asp
            180                 185                 190

Val Met Leu His Ala Gly Leu Thr Pro Glu Ala Ala Asn Asp Met Tyr
        195                 200                 205

Ala Tyr Glu Arg Asp Arg Ile Pro Thr His His Ile Ala His Ala Asp
    210                 215                 220

Glu Val Ala His Trp Ile Leu Arg Met Ala Asp Pro Ala Gly Arg His
225                 230                 235                 240

Ala Thr Gly Gln Val Ile Thr Val Asp Gly Gly Leu Glu Leu Ile
                245                 250                 255

<210>  21
<211>  756
<212>  DNA
<213>  Artificial synthesis

<400>  21
atgacgaagg tgactgttgt gacaggtgcg tcccgtgggc ttggcgaggc cattgtgaag      60

cagattctgg ccagaaatgc tgacgctaaa gtggtggccg ttgccagatc ggccgagaat     120

ttacagaagc tcgagaaagg ctcggagggt cgggttttgg ctgtggcagg cgatgtgacc     180

aggccagaga ccatacatag gctcattgag gagactgtgg caaaattcgg ccgaatcgac     240

tcggttgtgg tcaacgccgg cgttctagag cccgtccagc acattgattc gctcgacgtg     300

gacctcgtca ggcgtctcta cgaggtcaac ctgttctctg tgatggatct ggtgagacag     360

accttgcctt acatgcgtca atcaaaagga agttatctgt ttgtgtcgtc tggggcgtcc     420

accaagccct acgatgcctg gtctgcttac ggatcttcga aagcagcact caaccacttc     480

tgtttgagtc tggccaccga ggagccgctt atcagagctc tctccattgc acctggagtc     540

gtcgatacag acatgcagca ggatatccgc gaggtttttg ggcaaaatat ggccccagat     600

gccctcaaga ggtttacgga cttgcacgag aacaagcagc tgctggcccc tgaagtgccg     660

ggaggtttct atgcttcact ggcgctaaga ggagtcccag agaatttgaa tggtagatat     720

gtgagatatg atgattcaga gttgaaggag tacgcc                               756

<210>  22
<211>  252
<212>  PRT
<213>  Artificial synthesis

<400> 22

Met Thr Lys Val Thr Val Val Thr Gly Ala Ser Arg Gly Leu Gly Glu
1               5                   10                  15

Ala Ile Val Lys Gln Ile Leu Ala Arg Asn Ala Asp Ala Lys Val Val
            20                  25                  30

Ala Val Ala Arg Ser Ala Glu Asn Leu Gln Lys Leu Glu Lys Gly Ser
        35                  40                  45

Glu Gly Arg Val Leu Ala Val Ala Gly Asp Val Thr Arg Pro Glu Thr
    50                  55                  60

Ile His Arg Leu Ile Glu Glu Thr Val Ala Lys Phe Gly Arg Ile Asp
65                  70                  75                  80

Ser Val Val Val Asn Ala Gly Val Leu Glu Pro Val Gln His Ile Asp
                85                  90                  95

Ser Leu Asp Val Asp Leu Val Arg Arg Leu Tyr Glu Val Asn Leu Phe
            100                 105                 110

Ser Val Met Asp Leu Val Arg Gln Thr Leu Pro Tyr Met Arg Gln Ser
        115                 120                 125

Lys Gly Ser Tyr Leu Phe Val Ser Ser Gly Ala Ser Thr Lys Pro Tyr
    130                 135                 140

Asp Ala Trp Ser Ala Tyr Gly Ser Ser Lys Ala Ala Leu Asn His Phe
145                 150                 155                 160

Cys Leu Ser Leu Ala Thr Glu Glu Pro Leu Ile Arg Ala Leu Ser Ile
                165                 170                 175

Ala Pro Gly Val Val Asp Thr Asp Met Gln Gln Asp Ile Arg Glu Val
            180                 185                 190

Phe Gly Gln Asn Met Ala Pro Asp Ala Leu Lys Arg Phe Thr Asp Leu
        195                 200                 205

His Glu Asn Lys Gln Leu Leu Ala Pro Glu Val Pro Gly Gly Phe Tyr
    210                 215                 220

Ala Ser Leu Ala Leu Arg Gly Val Pro Glu Asn Leu Asn Gly Arg Tyr
225                 230                 235                 240

Val Arg Tyr Asp Asp Ser Glu Leu Lys Glu Tyr Ala
                245                 250

<210> 23
<211> 768
<212> DNA
<213> Artificial synthesis

<400> 23
atggctagga ccgcgctcgt caccggcgcc accagcggga tcggcctcgc cgtcgccaag 60

cagctcgccg aagccgggca tcaggtgttc ctctgcgccc gcaacgacgt ctcgccgacc 120

gtcaagcagc tgcgcgcgga cggcttcgag gtcgacggga ccgaatgcga cgtccggtcc 180

gagacggacg tccaggcgtt cgtgcaggca gcggtcgacc ggtacggccc ggtggacatc 240

ctggtcaaca acgcgggccg cagtggcggc ggggtcaccg ccgatctggt cgacgagctg 300

tggttcgacg tgatcgacac caacctgcac ggggtgttcc ggatgacccg cgcggtgctc 360

aacaccggcg ggctgcggca ccggccgtgg gggcggatcg tgaacatcgc gtcgaccgcg 420

ggcaagcagg gcgtggtgct gggcgcgccg tattcggcgt cgaagcacgc ggtcgtcggg 480

ttcaccaagt cattgggcaa cgagctggga aagacgggga tcacggtcaa cgcggtctgc 540

cctggttacg tcgaaacgcc gatggcgcag cgggtccggc agggttacgc ggccgcgtgg 600

gacatcagcg aggacgaggt gctcgaccgg ttccaggcca agatcccgct cggccggtac 660

agcaccccgg aggaggtcgc cggcctggtc ggctacctcg tctcggaccc ggccggctcg 720

atcaccgcgc aggcgctgaa cgtctgcggc ggcctcggca acttctga 768

<210> 24
<211> 255
<212> PRT
<213> Artificial synthesis

<400> 24

Met Ala Arg Thr Ala Leu Val Thr Gly Ala Thr Ser Gly Ile Gly Leu
1               5                   10                  15

Ala Val Ala Lys Gln Leu Ala Glu Ala Gly His Gln Val Phe Leu Cys
            20                  25                  30

Ala Arg Asn Asp Val Ser Pro Thr Val Lys Gln Leu Arg Ala Asp Gly
        35                  40                  45

Phe Glu Val Asp Gly Thr Glu Cys Asp Val Arg Ser Glu Thr Asp Val
    50                  55                  60

Gln Ala Phe Val Gln Ala Ala Val Asp Arg Tyr Gly Pro Val Asp Ile
65                  70                  75                  80

Leu Val Asn Asn Ala Gly Arg Ser Gly Gly Gly Val Thr Ala Asp Leu
                85                  90                  95

Val Asp Glu Leu Trp Phe Asp Val Ile Asp Thr Asn Leu His Gly Val
            100                 105                 110

Phe Arg Met Thr Arg Ala Val Leu Asn Thr Gly Gly Leu Arg His Arg
        115                 120                 125

Pro Trp Gly Arg Ile Val Asn Ile Ala Ser Thr Ala Gly Lys Gln Gly
    130                 135                 140

Val Val Leu Gly Ala Pro Tyr Ser Ala Ser Lys His Ala Val Val Gly
145                 150                 155                 160

Phe Thr Lys Ser Leu Gly Asn Glu Leu Gly Lys Thr Gly Ile Thr Val
                165                 170                 175

Asn Ala Val Cys Pro Gly Tyr Val Glu Thr Pro Met Ala Gln Arg Val
            180                 185                 190

Arg Gln Gly Tyr Ala Ala Ala Trp Asp Ile Ser Glu Asp Glu Val Leu
        195                 200                 205

Asp Arg Phe Gln Ala Lys Ile Pro Leu Gly Arg Tyr Ser Thr Pro Glu
    210                 215                 220

Glu Val Ala Gly Leu Val Gly Tyr Leu Val Ser Asp Pro Ala Gly Ser
225                 230                 235                 240

Ile Thr Ala Gln Ala Leu Asn Val Cys Gly Gly Leu Gly Asn Phe
                245                 250                 255

<210>  25
<211>  31
<212>  DNA
<213>  Artificial synthesis

<400>  25
ggtccactgg ccaatactcc tcctatcatc g                                    31


<210>  26
<211>  31
<212>  DNA
<213>  Artificial synthesis

<400>  26
cgatgatagg aggagtattg gccagtggac c                                    31


<210>  27
<211>  44
<212>  DNA
<213>  Artificial synthesis

<400>  27
gctttgatgc gggcgatggc aactacaagc gcatcgacat acag                      44


<210>  28
<211>  37
<212>  DNA
<213>  Artificial synthesis

<400>  28
gttgccatcg cccgcatcaa agccggtcgc gcaaatc                              37


<210>  29
<211>  64
<212>  DNA
<213>  Artificial synthesis

<400>  29
ggccatatga ataattttgt ttaactttaa gaaggagata taatgagtac caagatggat     60

tttg                                                                  64


<210>  30
<211>  32
<212>  DNA
<213>  ÈË¹¤ºÏ³É

<400>  30
cgcggatcct tacgcattag cctgctgttt gg                                    32

<210>  31
<211>  69
<212>  DNA
<213>  Artificial synthesis

<400>  31
ggcggatcca agcttaataa ttttgtttaa ctttaagaag gagatataat gacggatcgt      60

ctgaaaggc                                                              69

<210>  32
<211>  30
<212>  DNA
<213>  Artificial synthesis

<400>  32
ccgctcgagt tactgggcgg tatagccgcc                                       30